# EUROPEAN PATENT APPLICATION

(11) **EP 3 527 981 A2**
(43) Date of publication of application: **21.08.2019**
(21) Application number: 17862140.5
(22) Date of filing: 26.09.2017
(51) Int. Cl.: G01N 33/50, A61K 35/28

(54) **METHOD FOR SORTING HIGHLY EFFECTIVE STEM CELLS FOR TREATING IMMUNE DISORDER**

(30) Priority: 17.10.2016 KR 20160134085
(71) Applicant: Samsung Life Public Welfare Foundation, Seoul 04348 (KR)
(72) Inventor: YOO, Keon Hee, Seoul 06191 (KR); LEE, Myoung Woo, Seoul 05614 (KR); KIM, Dae Seong, Goyang-si Gyeonggi-do 10422 (KR)
(74) Representative: Gulde & Partner
(86) International application number: PCT/KR2017/010592
(87) International publication number: WO 2018/074758

(57) **Abstract**

The present invention relates to a method for sorting highly effective mesenchymal stem cells for treating an immune disorder, the method comprising a step of measuring the level of an immunosuppressive biomarker following IFN-γ pretreatment simulation in mesenchymal stem cells; highly effective mesenchymal stem cells sorted by the method; and a method for treating an immune disorder by using the highly effective mesenchymal stem cells. The present invention can provide a useful method capable of obtaining functionally excellent mesenchymal stem cells having an immune reaction regulation capability and used for clinically treating various immune disorders including graft-versus-host disease and autoimmune disorders, and thus can be usefully used as a therapy for immune disorders.

## Description

### [Technical Field]

The present invention relates to a method for sorting highly effective mesenchymal stem cells to treat an immune disorder, which includes measuring the level of an immunosuppressive biomarker after mesenchymal stem cells are stimulated by IFN-γ pretreatment, highly effective mesenchymal stem cells sorted by the method, and a method for treating an immune disorder using highly effective mesenchymal stem cells.

### [Background Art]

Conventionally, methods for transplanting allogeneic hematopoietic stem cells to treat malignant and non-malignant blood diseases, autoimmune diseases and immunodeficiency have been widely used. However, after the transplantation of human leucocyte antigen (HLA)-identical sibling, the onset of the immune disorder graft-versus-host disease (GVHD) and death caused thereby still remain a challenge.

GVHD is induced by a donor's T cells activated by antigen-presenting cells of a host, and the cells migrate to target tissues (the skin, intestines, and liver), thereby inducing the dysfunction of a target organ. The first-line standard therapy of the GVHD is to administer a high concentration of steroids. However, 50% of patients are not responsive to the first-line therapy, and once steroid resistance is induced, it is known that mortality increases, and there is no further therapeutic alternative because there is no second-line therapy for steroid-resistant GVHD.

Meanwhile, marrow stromal cells providing a growth factor, cellular interaction and a matrix protein are derived from common precursor cells present in the bone marrow microenvironment, and have been known as mesenchymal stem cells. Mesenchymal stem cells have differentiation capacity, and may produce precursor cells having a potential to differentiate into limited cells including osteoblasts, adipocytes and chondrocytes. Mesenchymal stem cells may replace host cells in the bone marrow microenvironment damaged by chemotherapy or radiotherapy, and may be used as a vehicle for gene therapy.

According to a variety of research, it has been reported that mesenchymal stem cells migrate to an injured region to restore damaged tissue, and have an immunomodulatory function. The mesenchymal stem cells activate and proliferate immune cells including T-cells, B-cells, natural killer cells (NK cells) and antigen-presenting cells, and downregulate the functions thereof. Immunosuppression mediated by mesenchymal stem cells may be achieved by soluble factors such as interleukin-10 (IL-10), TGF-β, nitrogen monoxide and prostaglandin E2 (PGE2).

Interferon-gamma (IFN-γ) is a potent pro-inflammatory cytokine that is produced from various types of cells such as activated T-cells, NK cells, NKT cells and macrophages, plays important and complicated roles in innate and adaptive immune responses, and has been known as a pathogenic factor involved in acute GVHD. IFN-γ negatively regulates alloreactive T-cells by inhibiting cell division and promoting cell death, and prevents tissue damage through direct interaction with a recipient's parenchymal cells.

As described above, through the previously performed *in vitro* research, the role of IFN-γ in activated mesenchymal stem cells has been reported, but little is known about the mechanism of treating *in vivo* GVHD by the transplantation of mesenchymal stem cells.

### [Disclosure]

### [Technical Problem]

Therefore, the inventors analyzed the effect of IFN-γ on expression profiles of biomarkers associated with the immunomodulatory function of mesenchymal stem cells through a microarray assay, investigated the action mechanism of mesenchymal stem cells associated with the control of immune conflicts *in vitro* and *in vivo,* and confirmed that mesenchymal stem cells derived from adipose tissue (AT), umbilical cord blood (CB), Wharton's jelly (WJ) and bone marrow, which are stimulated with IFN-y, increase the survival rate of GVHD models. Therefore, the present invention was completed.

Accordingly, the present invention is directed to providing a method for sorting highly effective mesenchymal stem cells to treat an immune disorder, which includes measuring the level of a particular immunosuppressive biomarker (IDO/CXCL9, CXCL10, CXCL11, ICAM1, ICAM2, B7-H1, PTGDS, VCAM1, or TRAIL) after stimulation by IFN-γ pretreatment, and highly effective mesenchymal stem cells sorted thereby.

However, technical problems to be solved in the present invention are not limited to the above-described problems, and other problems which are not described herein will be fully understood by those of ordinary skill in the art from the following descriptions.

### [Technical Solution]

The present invention provides a method for sorting highly effective mesenchymal stem cells to treat an immune disorder, which includes measuring the level of an immunosuppressive biomarker after mesenchymal stem cells are stimulated by IFN-γ pretreatment.

According to an exemplary embodiment of the present invention, the method includes the following steps:
(a) culturing mesenchymal stem cells and treating the cells with IFN-γ;
(b) measuring the expression level of an immunosuppressive biomarker in the mesenchymal stem cells; and
(c) determining the cells in which the expression level is increased compared with an IFN-γ-untreated control as highly effective mesenchymal stem cells for treating an immune disorder.

According to another exemplary embodiment of the present invention, the immunosuppressive biomarker is indoleamine 2,3-dioxygenase (IDO).

According to still another exemplary embodiment of the present invention, the immunosuppressive biomarker further includes one or more selected from the group consisting of C-X-C motif ligand 9 (CXCL9), C-X-C motif ligand 10 (CXCL10), C-X-C motif ligand 11 (CXCL11), InterCellular Adhesion Molecule 1 (ICAM1), InterCellular Adhesion Molecule 2 (ICAM2), B7 homolog 1 (B7-H1), Prostaglandin D2 synthase (PTGDS), Vascular Cellular Adhesion Molecule 1 (VCAM1) and TNF-Related Apoptosis-Inducing Ligand (TRAIL).

According to yet another exemplary embodiment of the present invention, the high efficiency is related to an immunosuppressive property.

According to yet another exemplary embodiment of the present invention, the mesenchymal stem cells are derived from any one selected from the group consisting of umbilical cord, umbilical cord blood, bone marrow, fat, muscle, Wharton's jelly, nerve, skin, amniotic membrane, chorion, decidua and placenta.

According to yet another exemplary embodiment of the present invention, the immune disorder is GVHD, rejection of organ transplantation, humoral rejection, an autoimmune disease or an allergic disease.

According to yet another exemplary embodiment of the present invention, the autoimmune disease is Crohn's disease, erythema, atopy, rheumatoid arthritis, Hashimoto's thyroiditis, malignant anemia, Edison's disease, Type I diabetes, lupus, chronic fatigue syndrome, fibromyalgia, hypothyroidism, hyperthyreosis, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma or ulcerative colitis.

According to yet another exemplary embodiment of the present invention, the IDO expression is increased in IFN-γ-stimulated mesenchymal stem cells through a JAK/STAT1 signaling pathway.

According to yet another exemplary embodiment of the present invention, IFN-γ in the step (a) is contained in a medium at a concentration of 1 to 100 IU/mL.

According to yet another exemplary embodiment of the present invention, the expression level of the biomarker in the step (b) is measured using western blotting, antibody immunoprecipitation, ELISA, mass spectrometry, RT-PCR, competitive RT-PCR, real-time RT-PCR, RNase protection assay (RPA), northern blotting or a DNA chip.

The present invention also provides highly effective mesenchymal stem cells for treating an immune disorder, which are sorted by the above-described method.

According to an exemplary embodiment of the present invention, the immune disorder is GVHD, rejection in organ transplantation, humoral rejection, an autoimmune disease or an allergic disease.

According to another exemplary embodiment of the present invention, the high efficiency is related to an immunosuppressive property.

According to still another exemplary embodiment of the present invention, the mesenchymal stem cells are derived from umbilical cord, umbilical cord blood, bone marrow, fat, muscle, Wharton's jelly, nerve, skin, amniotic membrane, or placenta.

According to yet another exemplary embodiment of the present invention, the mesenchymal stem cells may be derived from autologous, xenogeneic or allogeneic cells.

The present invention also provides a pharmaceutical composition for treating an immune disorder, which contains the highly effective mesenchymal stem cells.

The present invention also provides a pharmaceutical preparation for treating GVHD, which contains the highly effective mesenchymal stem cells.

The present invention also provides a method for treating an immune disorder such as GVHD, which includes administering the highly effective mesenchymal stem cells to a subject.

The present invention also provides a use of the highly effective mesenchymal stem cells for treating an immune disorder such as GVHD.

### [Advantageous Effects]

The present invention suggests that, after IFN-γ stimulation, a combination of particular biomarkers (IDO/CXCL9, CXCL10, CXCL11, ICAM1, ICAM2, B7-H1, PTGDS, VCAM1, and TRAIL) can be used as a biomarker for selecting mesenchymal stem cells having a most superior immunosuppressive property in immune-associated diseases including GVHD.

Accordingly, the present invention can provide a useful method capable of obtaining functionally superior mesenchymal stem cells having an ability to control an immune response for clinical treatment of various immune disorders including GVHD and an autoimmune disease, and can be effectively used as a therapeutic method for an immune disorder.

In addition, GVHD prevention and treatment using mesenchymal stem cells activated by IFN-γ shows higher therapeutic possibility of transplantation of allogeneic stem cells.

### [Description of Drawings]

FIGS. 1A to 1C show the morphology (FIG. 1A), surface antigenic proteins (FIG. 1B), and characterization after differentiation induction (FIG. 1C) of mesenchymal stem cells derived from various tissues (bone marrow (BM), adipose tissue (AT), cord blood (CB) and Wharton's jelly (WJ)).
FIG. 2 shows the result of evaluating a proliferation rate of peripheral blood mononuclear cells (hPBMCs) activated by PHA to confirm immunosuppressive characterization of mesenchymal stem cells derived from various tissues (BM, AT, CB and WJ).
FIGS. 3A to 3D show comparative evaluation results obtained by a direct contact culture method and a Transwell culture method to confirm whether the immunosuppressive properties of mesenchymal stem cells derived from various tissues (BM, AT, CB and WJ) are exhibited by an intercellular contact or a soluble factor.
FIGS. 4A to 4D show mouse survival rates after hPBMCs and mesenchymal stem cells derived from various tissues (BM, AT, CB and WJ) are injected into mice once or twice to confirm the immunosuppressive function of mesenchymal stem cells *in vivo.*
FIG. 5 shows the result of reducing an IFN-γ (inflammatory cytokine) level in blood when bone-marrow-derived mesenchymal stem cells are injected into a GVHD animal model together with hPBMCs.
FIG. 6 shows the result of comparing survival rates of the mesenchymal stem cells between an IFN-γ non-stimulated group (MSC^{PBS}) and a stimulated group (MSC^{IFN-γ}) to evaluate the survival rates after hPBMCs are cultured with mesenchymal stem cells derived from various tissues (BM, AT, CB and WJ).
FIGS. 7A to 7D show the results of comparatively evaluating mouse survival rates between groups to which no MSC, MSC^{PBS}, MSC^{IFN-γ}, and MSC^{AG490 + IFN-γ} were administered to confirm whether mesenchymal stem cells stimulated with IFN-γ improve GVHD.
FIG. 8 shows the result of comparatively measuring the numbers of CD45+ cells and CD45+CD3+ cells between groups to which no MSC, MSC^{PBS}, MSC^{IFN-γ}, and MSC^{AG490 + IFN-γ} were administered to confirm whether mesenchymal stem cells stimulated with IFN-γ induce the suppression of T cell proliferation in GVHD mouse models.
FIGS. 9A and 9B show the results of confirming that the penetration of immune cells into tissue is decreased through histological analysis after mesenchymal stem cells stimulated with IFN-γ are transplanted into a GVHD mouse model.
FIGS. 10A and 10B show the results of comparing the morphology (FIG. 10A) and gene expression profiles (FIG. 10B) of mesenchymal stem cells before and after IFN-γ stimulation.
FIG. 11 shows the result of confirming that mRNA expression levels of *CXCL9, CXCL10, CCL8* and *IDO* genes are significantly increased in mesenchymal stem cells stimulated with IFN-γ.
FIG. 12 shows the result of confirming that IDO expression is increased similarly when mesenchymal stem cells derived from various tissues (BM, AT, CB and WJ) are stimulated with IFN-γ.
FIGS. 13 and 14 show the results of confirming that IFN-γ-mediated IDO expression is induced through a JAK/STAT1 signaling pathway.
FIG. 15 shows the result that an hPBMC proliferation-inhibitory effect is interrupted by blocking a downstream signaling pathway of IFN-γ when *STAT1* target siRNA is treated.
FIG. 16 shows the immunohistochemical staining result of confirming the penetration of mesenchymal stem cells into tissue and the induction of IDO expression after the mesenchymal stem cells stimulated with IFN-γ are injected into GVHD mice.
FIGS. 17A to 17C show the results of confirming the change in hPBMC proliferation rate (FIG. 17C) after the suppression of IDO expression is confirmed by shRNA treatment (FIGS. 17A and 17B) to investigate the IDO role involved in the immunosuppressive property of mesenchymal stem cells.
FIG. 18 shows the result of comparatively evaluating a survival rate after IDO-expression-decreased mesenchymal stem cells, which are treated or not treated with IFN-γ, are administered into GVHD mice.
FIG. 19 shows the immunohistochemical staining result showing the presence of IDO-downregulated mesenchymal stem cells in small intestine and skin tissues after IDO-expression-decreased mesenchymal stem cells, which are treated or not treated with IFN-γ, are administered into GVHD mice.
FIG. 20 shows the result of confirming the immunosuppressive property of IDO-overexpressing mesenchymal stem cells *in vitro.*
FIG. 21 shows the result of confirming the immunosuppressive property of IDO-overexpressing mesenchymal stem cells *in vivo.*
FIG. 22 shows the immunohistochemical staining result showing the presence of IDO-upregulated mesenchymal stem cells in small intestine and skin tissues after IDO-overexpressing mesenchymal stem cells are administered into GVHD mice.
FIG. 23 shows the result of comparing immunosuppressive activities between mesenchymal stem cells stimulated with IFN-γ and TLR3-activated (poly I:C stimulation) mesenchymal stem cells.
FIGS. 24A and 24B show that, in mesenchymal stem cells stimulated with IFN-y, IDO expression is induced by a JAK/STAT1 pathway, but TLR3 activation is not induced.
FIG. 25 shows the RT-PCR result of comparatively evaluating the effect of mesenchymal stem cells on the expression of functional genes (*CXCL9, CXCL10, IL-*6 and *IL-8*) in IFN-γ stimulation and TLR3 activation (poly I:C stimulation).
FIG. 26 shows the RT-PCR result of comparatively evaluating the effect of mesenchymal stem cells on the expression of functional genes (IDO, CXCL9, CXCL10, CXCL11, ICAM1, ICAM2, B7-H1, PTGDS, VCAM1 and TRAIL) in IFN-γ stimulation, TNF-α stimulation, and TLR3 activation (poly I:C stimulation).

### [Modes of the Invention]

In a previous study, the inventors reported that activated T-cells expressed a higher level of IFN-γ than quiescent T-cells and that the IFN-γ expression level was significantly decreased when T-cells were co-cultured with mesenchymal stem cells, and suggested an autocrine-paracrine loop of IFN-γ. Therefore, in the present invention, it was expected that the stimulation of the mesenchymal stem cells by IFN-γ would improve the immunosuppressive property of cells, and a gene expression profile was analyzed to confirm whether the expression levels of various genes in the mesenchymal stem cells stimulated with IFN-γ are associated with the immunosuppressive property.

As a result, it was confirmed that the expression of 512 genes including *CXCL9, CXCL10, CCL8* and *IDO* playing critical roles in leukocyte recruiting causing various immune responses in the mesenchymal stem cells stimulated with IFN-γ is increased. Compared with these chemokines, IDO tends to be more directly involved in the immunosuppressive property of mesenchymal stem cells and is closely related to these activities. IDO was highly expressed in the mesenchymal stem cells stimulated with IFN-γ, and when IDO expression was inhibited, the antigen-mediated proliferation of T-cells was inhibited.

In addition, in the present invention, it was confirmed that IFN-γ can induce IDO expression even in mesenchymal stem cells derived from various tissues such as cord blood, adipose tissue and Wharton's jelly as well as human bone marrow. Particularly, it was seen that the IDO expression is increased in mesenchymal stem cells stimulated with IFN-γ through a JAK/STAT1 signaling pathway, and IDO-expressing mesenchymal stem cells exhibited an immunosuppressive property. The immunosuppressive activity of IDO is mediated by the degradation of tryptophan, which is an amino acid required for T-cell proliferation, and IDO and tryptophan deficiency is recognized as significant to various immune-associated diseases.

In addition, in the present invention, IDO expression in a GVHD mouse model into which mesenchymal stem cells stimulated with IFN-γ were injected was observed from images obtained by confocal microscopy, and the improved immunosuppressive activity of the mesenchymal stem cells was highly associated with IFN-γ pretreatment. It is expected that this result is caused by the induction of IDO expression.

In addition, in the present invention, it was confirmed that the survival rate of a group into which IFN-γ-stimulated mesenchymal stem cells were injected increased more than that of a group in which PBS (control)-treated mesenchymal stem cells were injected in GVHD mouse models, and thus it is considered the IFN-γ-stimulated mesenchymal stem cells will be effectively used as a cell therapy for GVHD.

In addition, in the present invention, as a result of confirming a signaling pathway involved in the increase in IDO expression in response to TLR signaling, TLR3 stimulation in human mesenchymal stem cells rarely induces IFN-β and/or IDO expression, which means that TLR signaling is not a critical pathway in terms of the immunosuppressive function of human mesenchymal stem cells.

In addition, since it was observed that IFN-γ stimulation significantly induces IDO expression in all mesenchymal stem cells, it can be seen that direct IFN-γ stimulation is a critical means for improving the function of mesenchymal stem cells to treat an immune-associated disease.

Therefore, the present invention provides a method for sorting highly effective mesenchymal stem cells to treat an immune disorder, which includes measuring the level of an immunosuppressive biomarker after the stimulation of IFN-γ pretreatment in mesenchymal stem cells.

The sorting method of the present invention may include: (a) treating with IFN-γ after culture of mesenchymal stem cells; (b) measuring the expression level of an immunosuppressive biomarker in the mesenchymal stem cells; and (c) determining the cells in which the expression level is increased compared with an IFN-γ-untreated control as highly effective mesenchymal stem cells for treating an immune disorder.

In the present invention, there is no limit to an immunosuppressive biomarker, which is preferably IDO, and more preferably, one or more selected from the group consisting of CXCL9, CXCL10, CXCL11, ICAM1, ICAM2, B7-H1, PTGDS, VCAM1 and TRAIL as well as the IDO marker.

The "high efficiency" used herein means effective efficiency in treatment of an immune-associated disease due to excellent immunosuppressive activity of mesenchymal stem cells.

The "mesenchymal stem cells (MSCs)" used herein refers to multipotent stem cells having an ability to differentiate into various mesodermal cells including bone, cartilage, adipose and muscular cells or ectodermal cells such as nerve cells. The MSCs are preferably derived from any one selected from the group consisting of umbilical cord, umbilical cord blood, bone marrow, fat, muscle, nerve, skin, amniotic membrane, chorion, decidua and placenta. In addition, the MSCs may be derived from a human, a fetus, or a non-human mammal. The non-human mammal is more preferably a canine animal, a feline animal, a simian animal, a cow, sheep, a pig, a horse, a rat, a mouse or a guinea pig, but the origin thereof is not limited.

The "immune disease" used herein is not limited as long as it is a disease caused by an immunomodulatory disorder, and may be, for example, graft-versus-host disease, rejection in organ transplantation, humoral rejection, an autoimmune disease or an allergic disease.

Here, the type of the autoimmune disease is not limited, and may be Crohn's disease, erythema, atopy, rheumatoid arthritis, Hashimoto's thyroiditis, malignant anemia, Edison's disease, Type I diabetes, lupus, chronic fatigue syndrome, fibromyalgia, hypothyroidism, hyperthyreosis, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma or ulcerative colitis.

Here, the type of the allergic disease is not limited, and may be anaphylaxis, allergic rhinitis, asthma, allergic conjunctivitis, allergic dermatitis, atopic dermatitis, contact dermatitis, urticaria, pruritus, insect allergy, food allergy or drug allergy.

In the present invention, in the MSCs stimulated with IFN-γ, IDO expression is increased through a JAK/STAT1 signaling pathway, which is a major information exchange pathway for cytokines, which are the key information exchange molecules in a hematopoietic or immune system. A signal transducer and activator of transcription 1 (STAT1) is tyrosine-phosphorylated by Janus kinase/Just another kinase (JAK), and thereby a dimer is formed, which then migrates to the nucleus and serves as a transcription factor (TF) for regulating gene expression. Therefore, IDO becomes a target gene of STAT1.

In the sorting method of the present invention, there is no limit to a concentration of IFN-γ with which an MSC culture solution is treated, and the concentration may be, for example, 1 to 100 IU/mL, preferably 1 to 10 IU/mL, and more preferably 1 IU/mL.

In the sorting method of the present invention, there is no limit to a method for measuring the expression level of a biomarker, and for example, protein expression may be measured using western blotting, antibody immunoprecipitation, ELISA, mass spectrometry, and mRNA expression may be measured using RT-PCR, competitive RT-PCR, real-time RT-PCR, RNase protection assay (RPA), northern blotting or a DNA chip.

In addition, the present invention provides highly effective MSCs for treating an immune disorder, which are sorted by the above-described method, and there is no limit to the origin of the MSCs. The cells may be, for example, autologous, xenogeneic or allogeneic cells.

In addition, the present invention provides a pharmaceutical composition/pharmaceutical preparation for treating an immune disorder, which contains the highly effective MSCs.

The "pharmaceutical composition" used herein may further include a component such as a conventional therapeutically active component, any of other additives, or a pharmaceutically acceptable carrier. Examples of the pharmaceutically acceptable carriers are saline, sterile water, Ringer's solution, buffered saline, a dextrose solution, a maltodextrin solution, glycerol, and ethanol.

The composition may be used by being formulated in the form of an oral preparation such as a powder, granules, a tablet, a capsule, a suspension, an emulsion, a syrup or an aerosol, a preparation for topical use, a suppository, or a sterile injection solution according to a conventional method.

A range of the "dose" used herein may be adjusted variously according to a patient's body weight, age, sex, health condition, diet, administration time, administration route, excretion rate, and severity of the disease, which is obvious to those of ordinary skill in the art.

The "subject" used herein refers to a subject in need of treatment, more specifically, a mammal such as a human, a non-human primate, a mouse, a rat, a dog, a cat, a horse or a cow.

The "pharmaceutically effective amount" used herein may be determined according to parameters including the type and severity of the disease for which administration of a drug is needed, the age and sex of a patient, the sensitivity to a drug, an administration time, an administration route, an excretion rate, a treatment period, a simultaneously used drug, and other parameters well known in the medical field, and it is an amount capable of obtaining the maximum effect without side effects in consideration of all parameters, and may be easily determined by those of ordinary skill in the art.

There is no limit to the "administration route" as long as the composition of the present invention can reach target tissue. Examples of the administration route include oral administration, intraarterial injection, intravenous injection, percutaneous injection, intranasal administration, transbronchial administration, and intramuscular administration. A daily dose may be about 0.0001 to 100 mg/kg, and preferably 0.001 to 10 mg/kg, and the administration is preferably performed once to several times a day.

Hereinafter, to help in understanding the present invention, exemplary examples will be suggested. However, the following examples are merely provided so that the present invention can be more easily understood, and not to limit the present invention.

### [Examples]

### 1. Experimental methods

### 1-1. Isolation and culture of human tissue-derived MSCs

The experiment was approved (IRB No. 2011-10-134) by the Institutional Review Board (IRB) of Samsung Medical Center, and all samples were obtained with informed consent. Bone-marrow-derived MSCs (BM-MSCs), cord-blood-derived MSCs (CB-MSCs), adipose-tissue-derived MSCs (AT-MSCs), and Wharton's-jelly-derived MSCs (WJ-MSCs) were isolated by a conventionally known method. The isolated cells were seeded at a density of 2×10³ cells/cm² in Dulbecco's Modified Eagle's Medium (DMEM; Invitrogen-Gibco, Rockville, MD) containing 10% fetal bovine serum (FBS, Invitrogen-Gibco) and 100 U/mL penicillin/streptomycin (Invitrogen-Gibco), and cultured under conditions of 37 °C and 5% CO₂.

### 1-2. T-cell proliferation: BrdU incorporation assay

MSCs were seeded at a density of 1.25×10⁴ cells/mL in a 96-well plate using 10% FBS-supplemented high-glucose DMEM (Invitrogen-Gibco, Rockville, MD). After 24 hours, to suppress the cell proliferation, 10 µg/mL of mitomycin-C (Sigma-Aldrich, St. Louis, MO) was added, and then the cells were further cultured at 37 °C for 2 hours and washed with a culture medium five times. Subsequently, 1×10⁵ human peripheral blood-derived mononuclear cells (hPBMCs) were isolated by density gradient centrifugation, added to each well, and treated with 1 µg/mL of phytohemagglutinin (PHA, Sigma-Aldrich) to promote T-cell proliferation. The hPBMCs activated by PHA treatment were cultured together with MSCs under different conditions for 3 to 4 days before the addition of 5-bromo-2-deoxyuridine (BrdU). A T-cell proliferation rate was assessed using Roche Applied Science(Penzberg, Germany) after 18 hours of treatment with BrdU.

### 1-3. GVHD animal model

300 cGy of total body irradiation was performed on 8- to 9-week-old NOD/SCID immunodeficient mice (Jackson Laboratories, Bar Harbor, ME), and after 24 hours, hPBMCs were intravenously administered. More specifically, 2×10⁷ hPBMCs were injected into each mouse together with 1×10⁶ MSCs. Here, the MSCs which were or were not stimulated with IFN-γ were used. Afterward, the same number of MSCs were injected again 7 days after the injection.

### 1-4. Inhibition of JAK and STAT1 activities

The inhibition of the activity of Janus kinase (JAK) corresponding to an intracellular domain of an IFN-γ receptor was performed by treating with 100 ng/mL of an anti-IFN-γ antibody (BD Biosciences) or 1 µM AG490 (Calbiochem, San Diego, CA), the inhibition of signal transducer and activator of transcription 1 (STAT1) expression was performed using siRNA (Santa Cruz Biotechnology) targeting an STAT1 gene, and cells were cultured with a siRNA-Lipofectamine 2000 (Invitrogen-Gibco) complex at 37 °C for 12 hours.

### 1-5. Immunoblotting

MSCs were washed with cold PBS and lysed with 300 µl of cold RIPA buffer (50 mM Tris-HCl, pH 7.5, containing 1% Triton X-100, 150 mM NaCl, 0.1% sodium dodecyl sulfate (SDS), 1% sodium deoxycholate, and a protease inhibitor cocktail (Thermo Fisher Scientific, Rockford, IL)). Subsequently, a cell lysate was centrifuged at 4 °C and 3,000×g for 15 minutes, and the supernatant was collected to perform protein quantitative analysis using a bicinchoninic acid protein assay kit (Thermo Fisher Scientific).

Subsequently, for electrophoresis, a protein (50 µg) was dissolved in a sample buffer (60 mM Tris-HCl, pH 6.8, containing 14.4 mM β-mercaptoethanol, 25% glycerol, 2% SDS, and 0.1% bromophenol blue), boiled for 5 minutes, and loaded in a 4 to 12% SDS reducing gel to separate proteins by size. The separated protein was transferred onto a polyvinylidene difluoride (PVDF) membrane (GE Healthcare, Buckinghamshire, UK) using a trans-blot system (Invitrogen-Gibco). Afterward, membrane blots were treated with Tris-buffered saline (TBS) (10 mM Tris-HCl, pH 7.5, supplemented with 150 mM NaCl) containing 5% non-fat dry milk (BD Biosciences) and reacted for 1 hour at room temperature, to thereby perform blocking. The blots were washed with TBS three times, and treated at 4 °C overnight with primary antibodies diluted in TBST (TBS supplemented with 0.01% Tween 20) containing 3% non-fat dry milk. The following day, the membrane blots were washed with TBST three times, treated with secondary antibodies diluted in 3% TBST containing non-fat dry milk, and then reacted at room temperature for 1 hour. Afterward, the membrane blots were washed with TBST again, and then the expression level of a protein to be observed was analyzed using an enhanced chemiluminescence detection system (GE Healthcare).

Here, antibodies specific for phospho-JAK 2 (#3771), STAT1 (#9172), phospho-STAT1 (#9171) and β-actin (#4967) were purchased from Cell Signaling Technology (Danvers, MA), and antibodies specific for IDO (sc-25808) and IRF-1 (sc-13041) were purchased from Santa Cruz Biotechnology.

### 1-6. Establishment of genetically modified MSCs

To inhibit IDO expression, human IDO shRNA lentivirus particles and IDO expression-induced lentivirus particles were purchased from Santa Cruz Biotechnology (sc-45939-V; Santa Cruz, CA) and GenTarget Inc. (LVP302; San Diego, CA), respectively. First, in order to transfect bone-marrow-derived stem cells with lentivirus particles for inhibiting IDO expression, the cells were pretreated with 5 µg/mL of polybrene (Santa Cruz Biotechnology) prepared using 10% FBS-added LG-DMEM, and then treated with a lentivirus vector at a multiplicity of infection (MOI) of 10.

Afterward, the cells were cultured for 24 hours under conditions of 37 °C and 5% CO₂, and washed with phosphate-buffered saline (PBS; Biowest, Nuaille, France) twice, and then a medium was added thereto again. One day after the introduction of the lentivirus-vector-introduced MSCs, the cells were cultured in an MSC medium containing 5 µg/mL of puromycin (Sigma-Aldrich) for 7 days and sorted, and then western blotting was performed. Meanwhile, to transfect to the bone-marrow-derived MSCs with the IDO-expression-induced lentivirus particles, the cells were treated with the lentivirus particles at an MOI of 10 using 10% FBS-added LG-DMEM, cultured for 72 hours under conditions of 37 °C and 5% CO₂, and washed with PBS twice, and then a culture medium was added again.

Subsequently, one day after introduction, the MSCs into which the lentivirus vector was introduced were cultured in an MSC medium containing 5 µg/mL of puromycin (Sigma-Aldrich) for 7 days and then sorted by the same method as described above, and then fluorescent observation using a red fluorescent protein (RFP) and western blotting were performed.

### 1-7. Immunocytochemistry and immunohistochemistry

MSCs were treated with 4% formaldehyde as a fixative solution, reacted at room temperature for 30 minutes while light was blocked, and washed with PBS three times. To detect a protein expressed in cells, the cells were treated with 0.25% Triton X-100, and reacted at room temperature for 5 minutes while light was blocked to increase a cell penetration property. Afterward, the cells were washed three times again, treated with a 5% FBS blocking solution, reacted at room temperature for 1 hour, washed again, treated with IDO-specific antibodies (1:100) purchased from Santa Cruz Biotechnology (Santa Cruz, CA), and reacted at room temperature for 1 hour. Subsequently, the cells were washed again three times and reacted with Alexa Fluor®488-conjugated goat anti-mouse IgG (Invitrogen-Gibco) secondary antibodies at room temperature for 1 hour, and then cell images were obtained using Carl Zeiss LSM 700 confocal microscope system (Jena, Germany).

MSCs were treated with trypsin, incubated using 1 µM CM-DiI CellTracker (Invitrogen-Gibco) at 37 °C for 5 minutes, and then further incubated at 4 °C for 15 minutes. Subsequently, 1×10⁶ labeled MSCs were washed with PBS, intravenously administered into a mouse together with hPBMCs, and after 7 days, the same number of cells were administered again. Afterward, the mouse was sacrificed to separate the small intestine and cut with a frozen sectioning technique, and then the tissues were washed twice and then reacted with the cells at room temperature for 1 hour after a 5% FBS blocking solution was added. The tissue was washed once more in the same manner as described above, and primary antibodies against IDO (1:100) were treated and reacted with cells at room temperature for 1 hour, and then fluorescence images were obtained from the tissue using a Carl Zeiss LSM 700 confocal microscopy system. From the images, a nucleus (blue; Vector Laboratories, Burlingame, CA), IDO (green), and CM-DiI-labeled MSCs (red) were detected. The confocal microscope images were analyzed using LSM 700 Zen software.

### 1-8. RT-PCR analysis

MSCs were cultured for 24 hours in the presence or absence of 200 IU/mL of IFN-γ and/or 100 µg/mL of poly I:C (Invitrogen-Gibco), and then total RNA was extracted using a QIAGEN RNeasy Mini Kit (QIAGEN, Valencia, CA), and used to perform a semi-quantitative reverse transcription-polymerase chain reaction (RT-PCR) using a PrimeScript™ 1st strand cDNA synthesis kit (TaKaRa Shuzo, Shiga, Japan), thereby synthesizing cDNA.

Primer sequences used in the PCR are as follows.
IDO forward: 5'-GCGCTGTTGGAAATAGCTTC-3'
IDO reverse: 5'-CAGGACGTCAAAGCACTGAA-3'(234 bp)
IFN-γ forward: 5'-TTGGCTTTTCAGCTCTGCATC-3'
IFN-γ reverse: 5'-GGAGACAATTTGGCTCTGCATT-3'(201 bp)
GAPDH forward: 5'-TCAACGGATTTGGTCGTATTGGG-3'
GAPDH reverse: 5'-TGATTTTGGAGGGATCTCGC-3'(234 bp)

### 1-9. Statistical analysis

All experimental results were expressed as mean ± standard deviation, and the difference in each experimental condition was analyzed using *t*-test or analysis of variance. When the P-value was less than 0.05, it was considered statistically significant.

### 2. Experimental results

### 2-1. Analysis of characterization of human tissue-derived MSCs

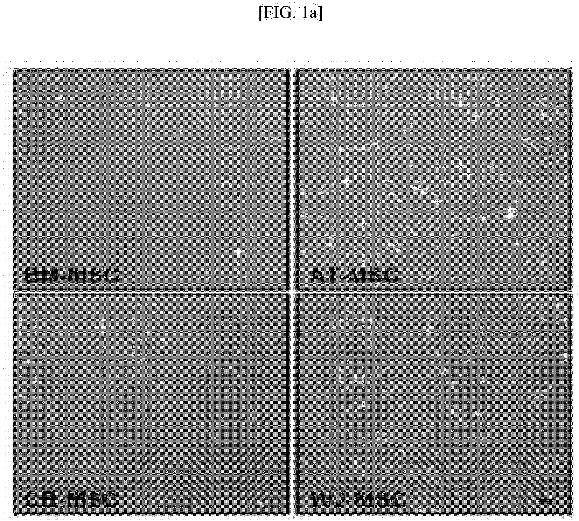

As a result of observing human MSCs isolated from bone marrow, cord blood and Wharton's jelly, it was confirmed that the cells were fibroblastic in shape, whereas it was observed that adipose-tissue-derived MSCs had a small spindle-like shape (FIG. 1A).

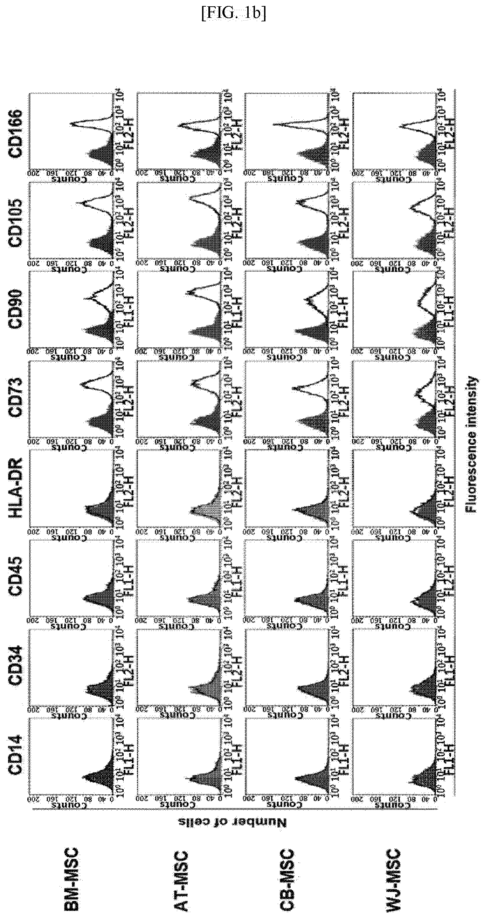

In addition, as a result of confirming an antigen protein expressed on a surface through cell analysis, it was confirmed that all of the cells expressed CD73, CD90, CD105 and CD166, and none expressed hematopoietic stem cell-derived markers such as CD14, CD34, CD45 and HLA-DR (FIG. 1B).

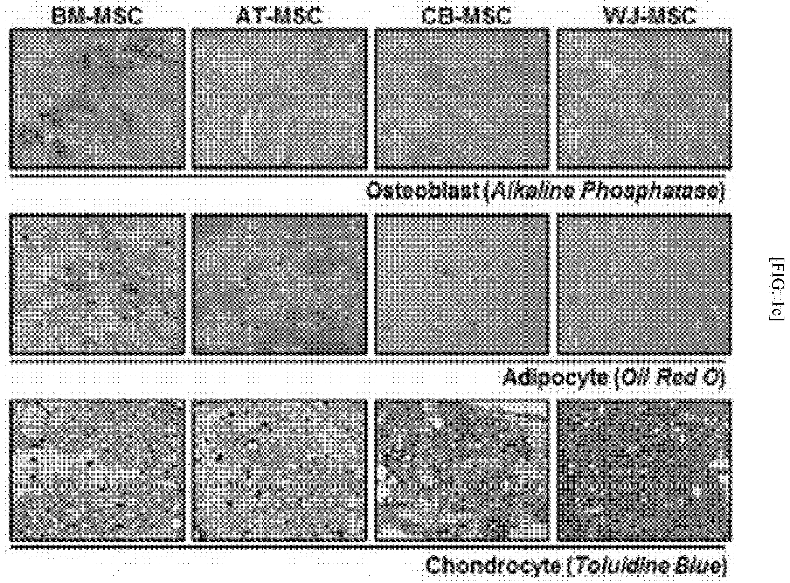

In addition, the MSCs were cultured for 14 to 21 days in each of osteogenesis-, adipogenesis- and chondroplasia-inducing media, and thereby alkaline phosphatase activity, the accumulation of triglyceride vacuoles and the accumulation of a chondrocyte matrix were observed, respectively. It was confirmed that all MSCs show osteocyte-, adipocyte- and chondrocyte-like morphologies regardless of their origins when the cells were cultured in suitable media and differentiation was induced (FIG. 1C).

### 2-2. Confirmation of immunosuppressive property of MSCs in in vitro and in vivo models

The immunosuppressive property of naive MSCs was evaluated using a mixed lymphocyte reaction (MLR).

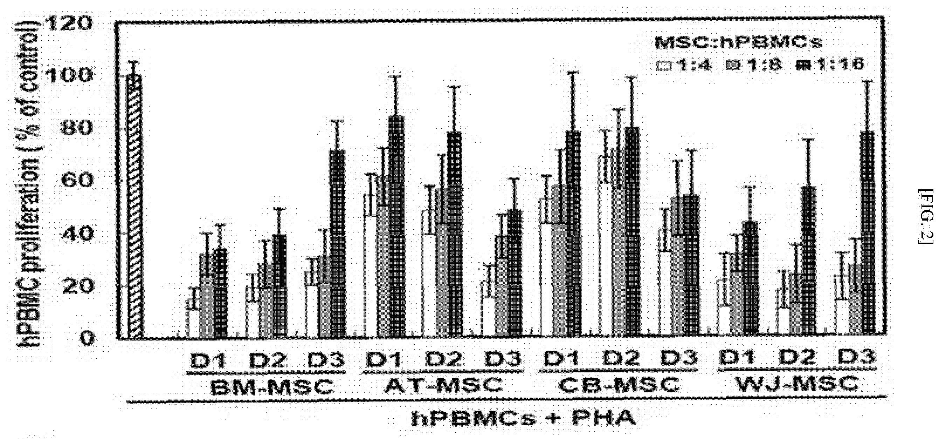

First, as a result of culturing PBMCs activated by treatment with PHA on MSCs derived from bone marrow, adipose tissue, cord blood or Wharton's jelly-, T-cell activity was significantly decreased, confirming that the result is dependent on the number of MSCs in co-culture (FIG. 2). In addition, there was no significant difference in an immunomodulatory property of MSCs derived from different tissue, which means that MSCs derived from adipose tissue, cord blood and Wharton's jelly were effective in inhibiting T-cell proliferation like bone-marrow-derived MSCs.

Subsequently, to investigate whether the inhibition of T-cell proliferation by the MSCs was directly mediated by the MSCs or caused by a soluble factor, PBMCs were co-cultured with MSCs in a Transwell insert using a Transwell system.

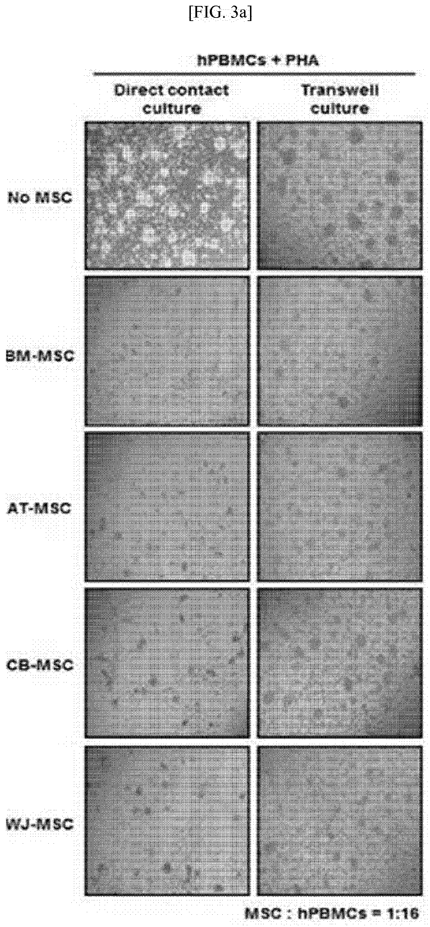

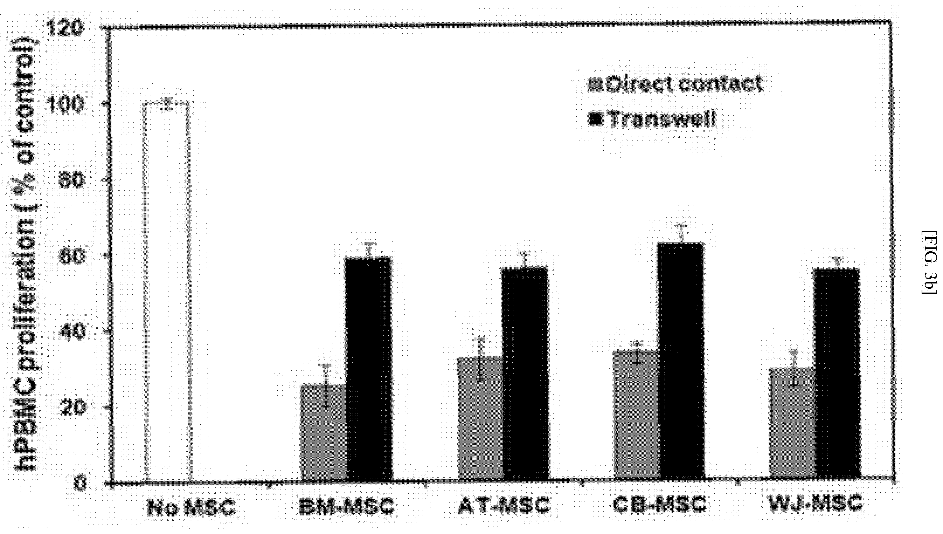

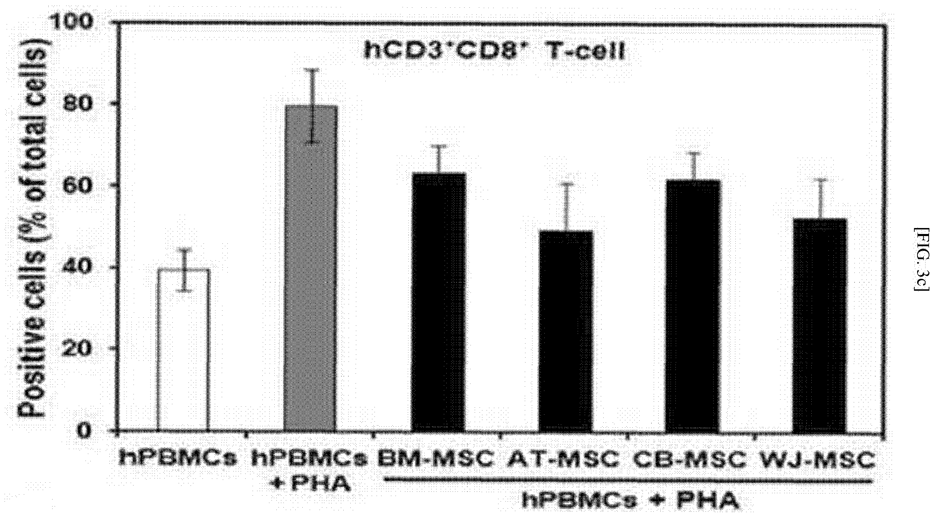

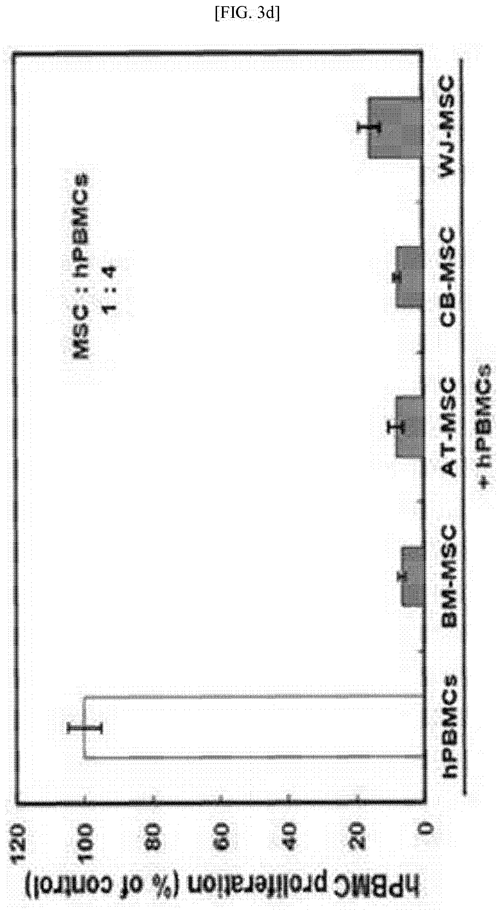

As a result, as shown in FIG. 3, the MSCs derived from each tissue inhibited hCD3+CD8+ T-cell proliferation in response to PHA stimulation without cell contact, which was somewhat decreased compared with a proliferation inhibitory level exhibited under the condition of direct contact between cells. The results mean that, in addition to the direct contact between cells, a solubility factor also contributes to the immunomodulatory effect of MSCs.

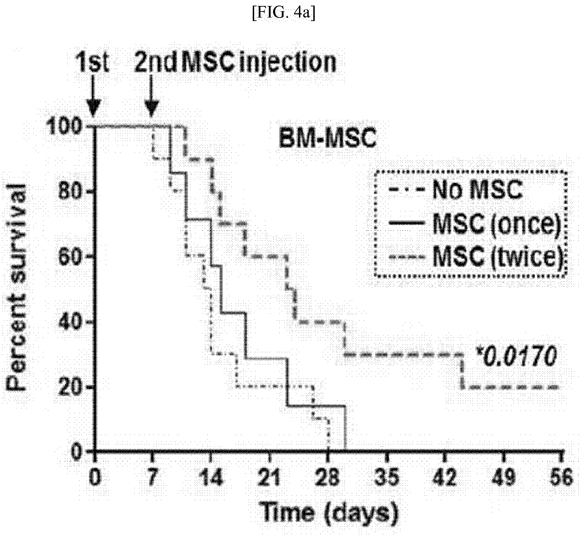

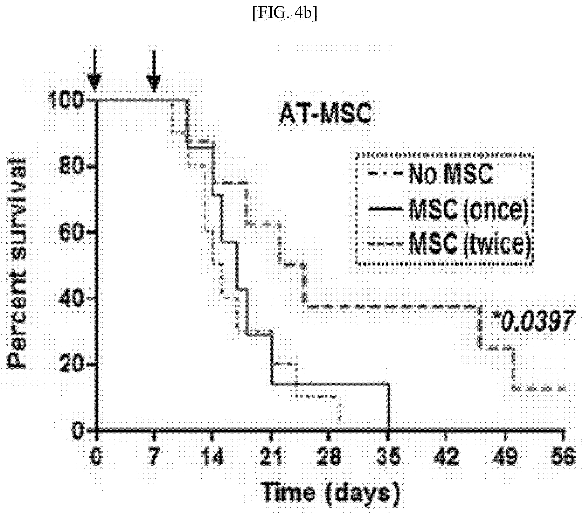

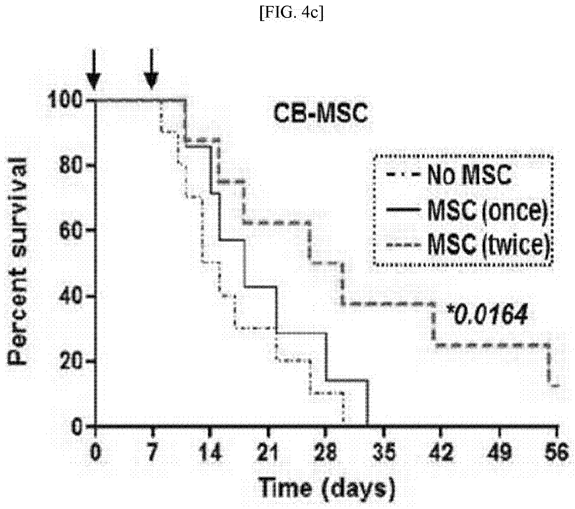

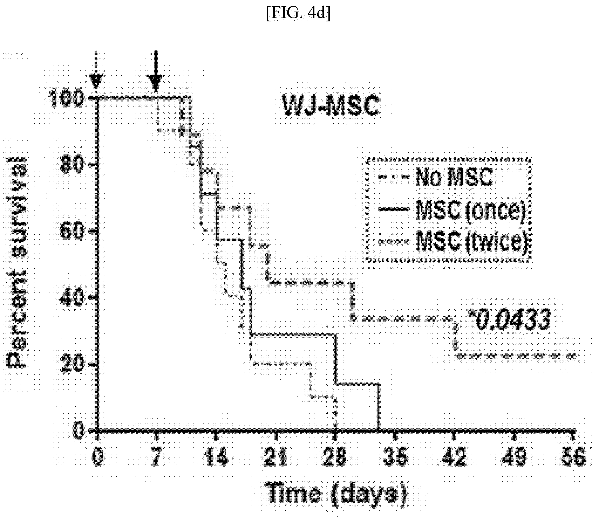

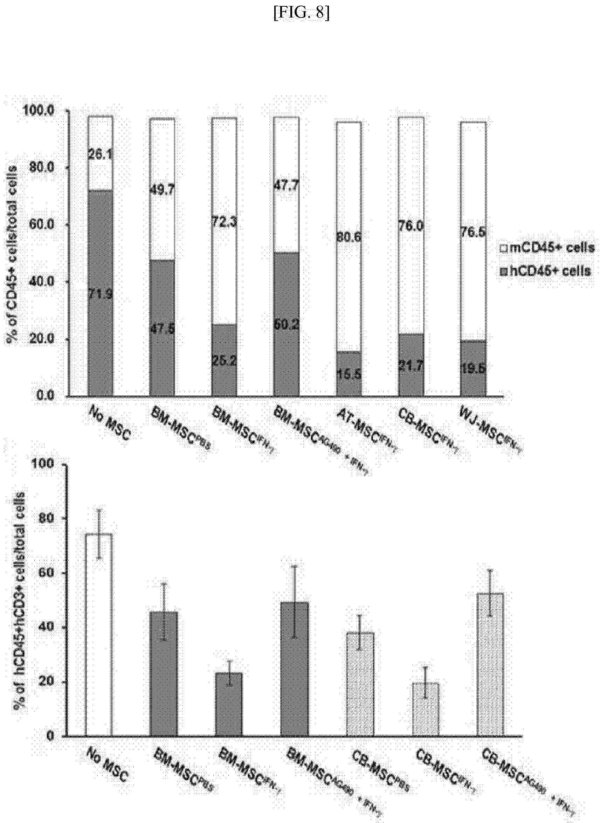

In addition, to confirm the immunosuppressive function of MSCs *in vivo,* mice were injected with PBMCs and MSCs derived from different tissues, and as shown in FIG. 4, 8 weeks after the cell injection, the mice injected with hPBMCs alone, or once with a combination of the cells and MSCs died, but about 20% of the mice injected twice with MSCs survived.

In addition, to investigate the role of a solubility factor in immunosuppression, a supernatant of a mitogen-activated T-cell culture cultured in the presence or absence of MSCs was analyzed. As a result, when PBMCs were activated, the secretion of various cytokines, chemokines, and growth factors was increased. Meanwhile, when the MSCs were present, the amounts of inflammatory cytokines such as IFN-γ and TNF-α were decreased regardless of their origins.

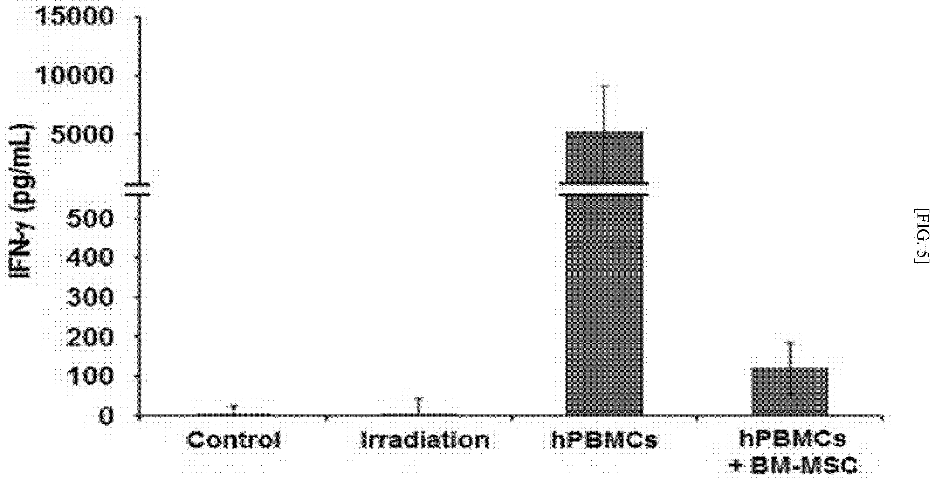

In addition, similar to the *in vitro* result, even when PBMCs and the bone-marrow-derived MSCs were injected into GVHD animal models, it was confirmed that a blood IFN-γ level was significantly reduced (FIG. 5).

### 2-3. Confirmation of improvement in immunosuppressive property of MSCs by IFN-γ stimulation

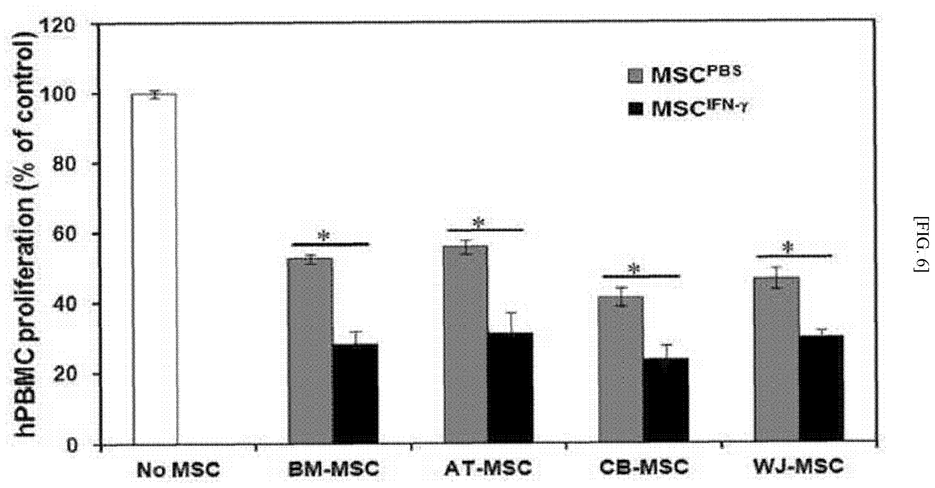

When PBMCs were co-cultured with the MSCs, proliferation was inhibited, and when PBMCs were co-cultured with MSCs stimulated with IFN-γ, it was confirmed that proliferation was further inhibited (FIG. 6).

Therefore, to examine whether the IFN-γ-stimulated MSCs can improve GVHD, IFN-γ-stimulated MSCs were injected twice into the hPBMC-injected mice at an interval of 7 days. Afterward, the mouse survival rates in a group into which only PBMCs were injected, a group into which PBMCs and MSCs were co-injected, a group into which PBMCs and IFN-γ-stimulated MSCs were co-injected, and a group into which a JAK inhibitor AG490 was pretreated, and then PBMCs and IFN-γ-stimulated MSCs were co-injected were compared.

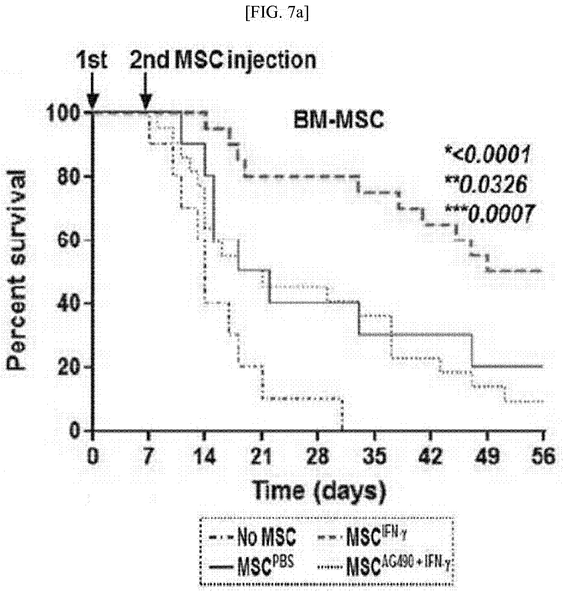

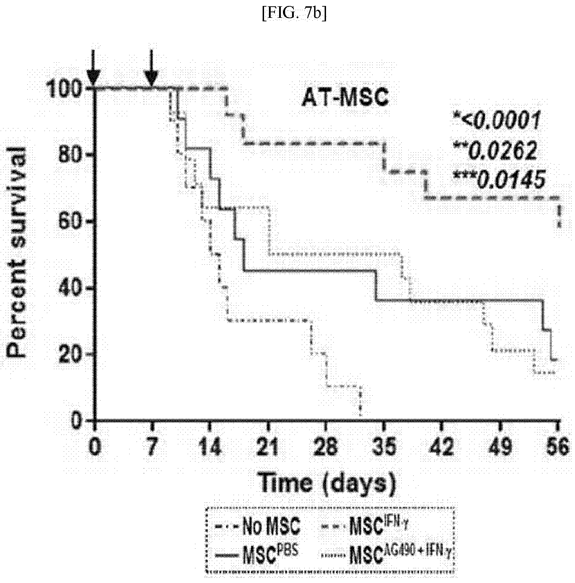

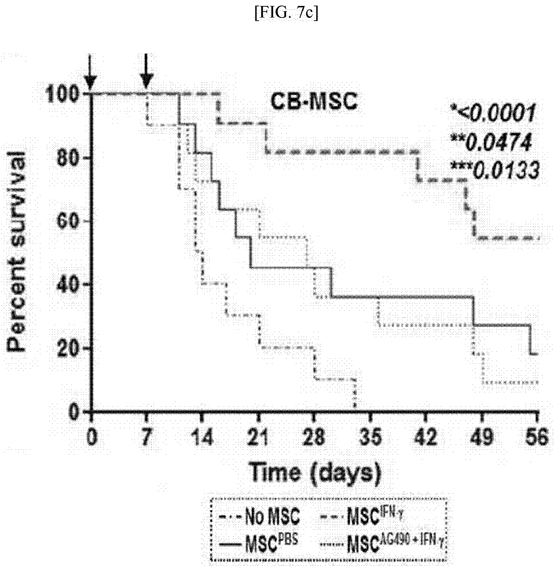

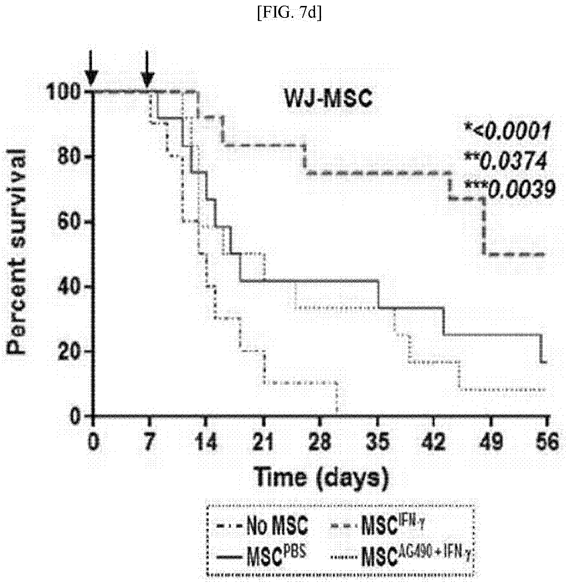

As a result, as shown in FIG. 7, compared with the mice into which PBMCs were injected alone, it was confirmed that the survival rate of the mice into which a combination of PBMCs and IFN-γ-stimulated MSCs was injected was improved. In addition, the improvement in survival rate was much higher than that when a combination of PBMCs and IFN-γ-untreated MSCs was injected. However, it was confirmed that, when a JAK inhibitor was pretreated, the improvement in survival rate of GVHD mice due to the IFN-γ-stimulated MSCs was reduced.

In addition, to confirm whether the IFN-γ-stimulated MSCs induced the inhibition of T-cell proliferation in a GVHD mouse model, blood in each mouse group was analyzed by flow cytometry, and as shown in FIG. 8, it was confirmed that, when a combination of PBMCs and MSCs was injected, the numbers of CD45+ and CD45+CD3+ cells were decreased, but when PBMCs were injected together with the IFN-γ-stimulated MSCs, the number of CD45+ and CD45+CD3+ cells were much greatly decreased.

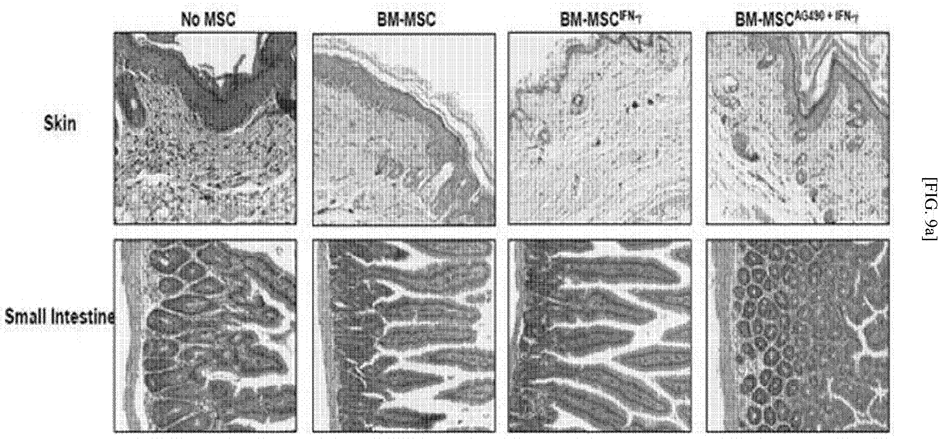

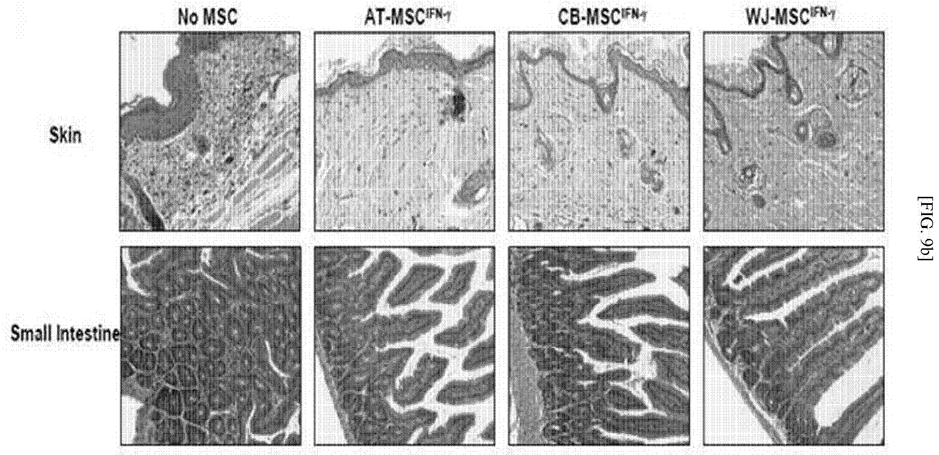

In addition, through histological analysis, as shown in FIG. 9, it was confirmed that, when PBMCs were injected together with the IFN-γ-stimulated MSCs, the clinical symptoms of a GVHD mouse and the penetration of immune cells were effectively decreased in the skin and small intestine. This result means that the IFN-γ stimulation improved the immunosuppressive property of MSCs.

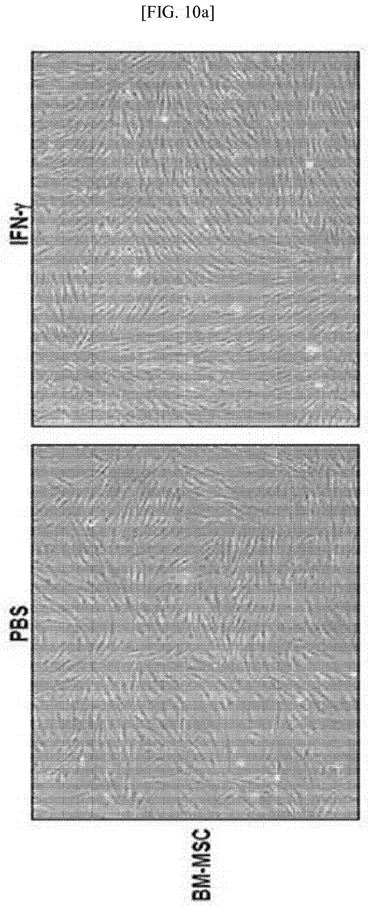

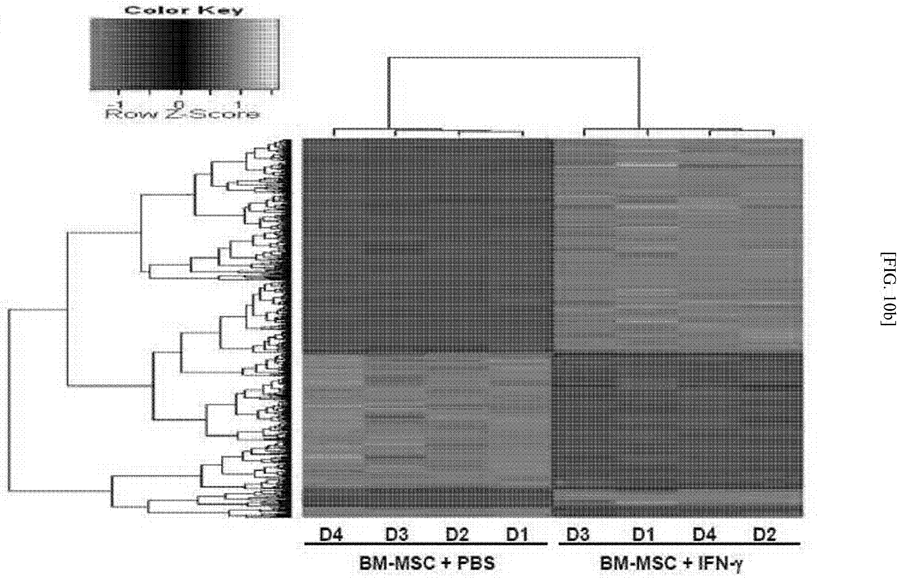

Subsequently, to investigate the effect of IFN-γ on the gene expression of MSCs, before and after IFN-γ stimulation, a gene expression profile of the MSCs was compared. As a result, when the bone-marrow-derived MSCs were stimulated with IFN-γ, it was confirmed that there was no significant difference in morphological change (FIG. 10A), and there was a large difference in gene expression profile (FIG. 10B).

Actually, the expression of 512 genes was increased in the IFN-γ-stimulated MSCs, and referring to Table 1 below, it can be seen that four of the genes are associated with the immunosuppressive function of MSCs.

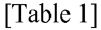

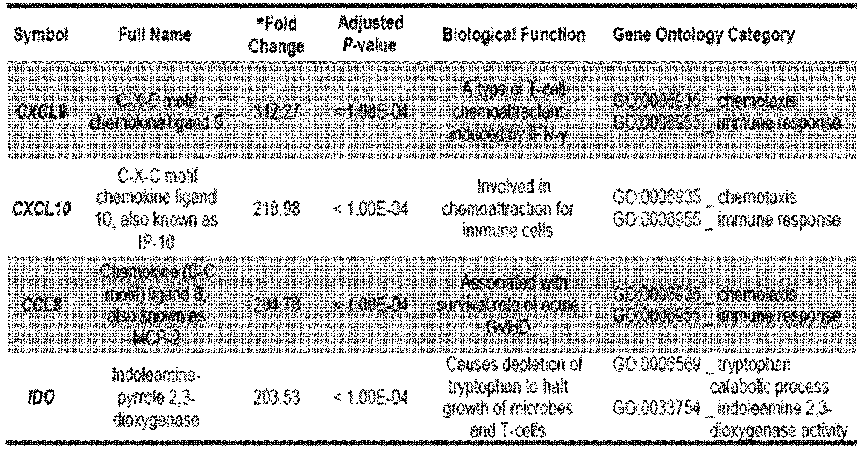

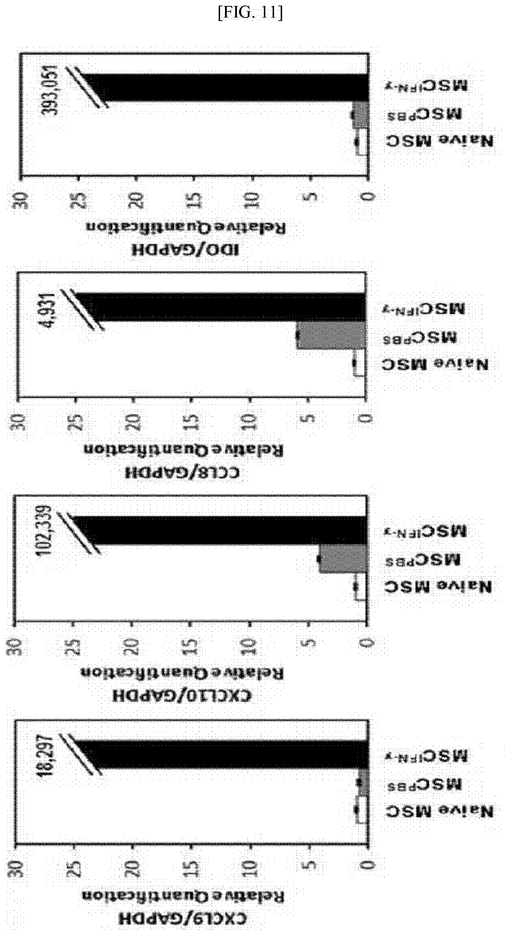

In addition, through qRT-PCR, as shown in FIG. 11, it was confirmed that mRNA expression levels of *CXCL9* (C-X-C motif), *CXCL10, CCL8* (C-C motif) and *IDO* genes were significantly increased in the IFN-γ-stimulated MSCs.

### 2-4. Confirmation of IFN-γ mediated induction of IDO expression via JAK/STAT1 signaling pathway

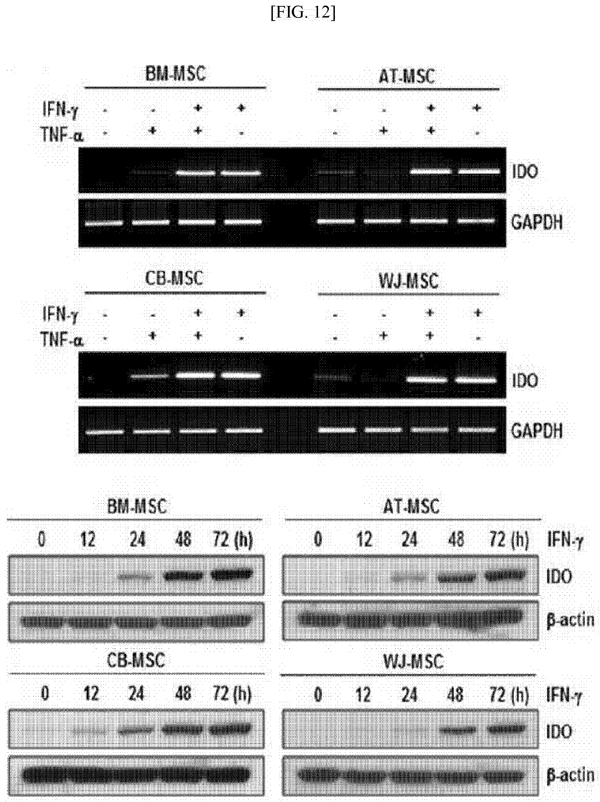

From the above results, it was confirmed that, when bone-marrow-derived MSCs were stimulated with IFN-γ, IDO expression was increased, and the same result was shown in MSCs derived from different tissues (FIG. 12).

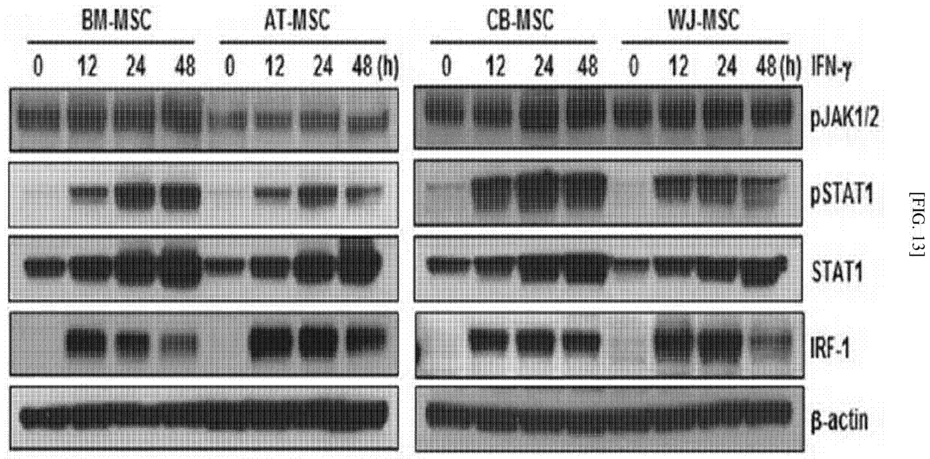

IFN-γ signaling occurs via a JAK/STAT1 pathway, and STAT1 is phosphorylated, migrates to a nucleus, and mediates transcription. Therefore, according to immunoblotting analysis, as shown in FIG. 13, compared with MSCs that were not stimulated with IFN-γ, STAT1 activation (STAT1 phosphorylation) in the IFN-γ-stimulated cells was confirmed.

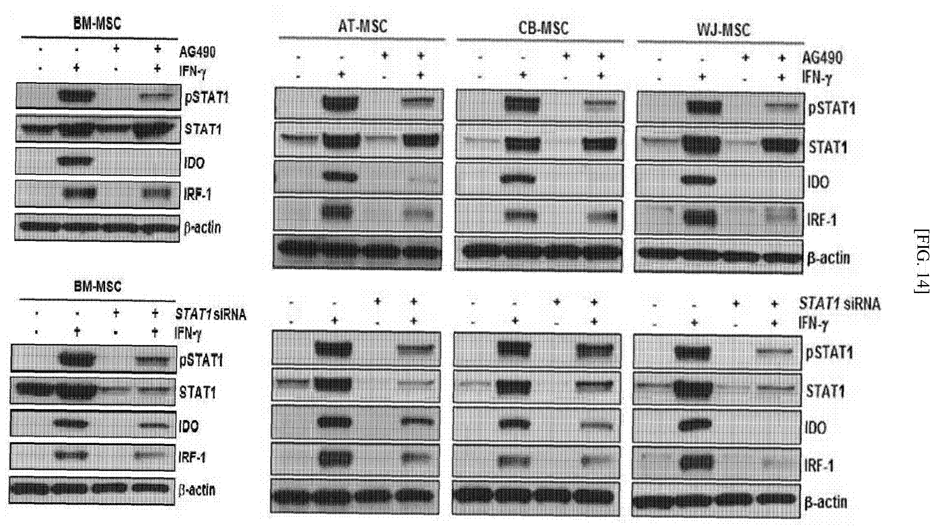

In addition, to examine the correlation between the JAK/STAT1 pathway and the IDO expression, JAK inhibitor (AG490) or siRNA targeting *STAT1* treatment was performed and then immunoblotting was performed, and therefore, as shown in FIG. 14, when the JAK inhibitor or siRNA targeting *STAT1* treatment was performed, it was confirmed that little to no IDO was expressed. This means that the IFN-γ-induced increase in IDO expression occurs via the JAK/STAT1 pathway.

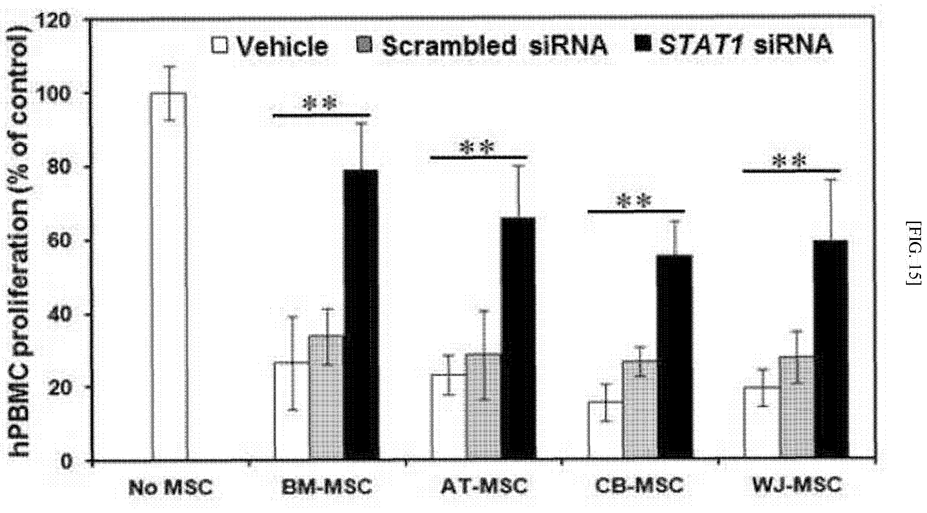

The MLR result showed that MSCs inhibit PBMC proliferation, but confirmed that this effect was not shown by the treatment with an anti IFN-γ antibody, demonstrating that IFN-γ secreted by activated immune cells including T-cells plays a very critical role for the immunomodulatory property of MSCs due to IDO expression. In addition, this result can be confirmed by the inhibition of the downstream signaling pathway of IFN-γ through the treatment of siRNA targeting *STAT1* (FIG. 15).

Consequently, it can be seen that the induction of IDO expression in MSCs via the IFN-γ/JAK/STAT1 pathway is very critical for the immunosuppressive function of the MSCs.

In addition, the *IDO* mRNA expression was increased when the MSCs were treated with IFN-γ at least for four hours. It was confirmed that, when they were treated with 1 IU/mL of IFN-γ for 24 hours, the *IDO* mRNA expression was maintained at least for 1 day, and the IDO protein level was maintained at least for 7 days. In addition, when the same MSCs were retreated with 1 IU/mL of IFN-γ, *IDO* mRNA was re-expressed, confirming that a protein level increased more.

### 2-5. Identification of IDO role in immunosuppressive property of human MSCs

To evaluate whether MSCs injected into a GVHD mouse were present in tissue of the mouse, CM-DiI-stained MSCs were injected, and then observed using a confocal microscope.

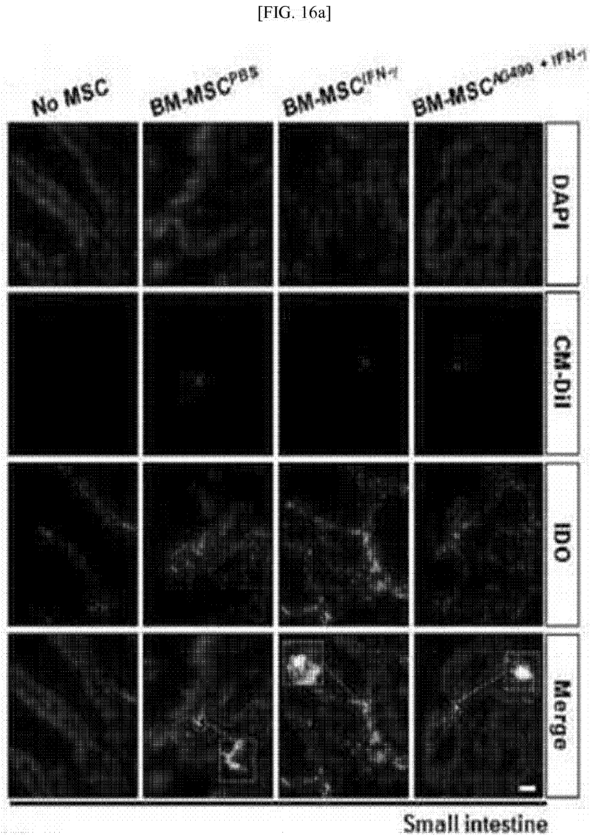

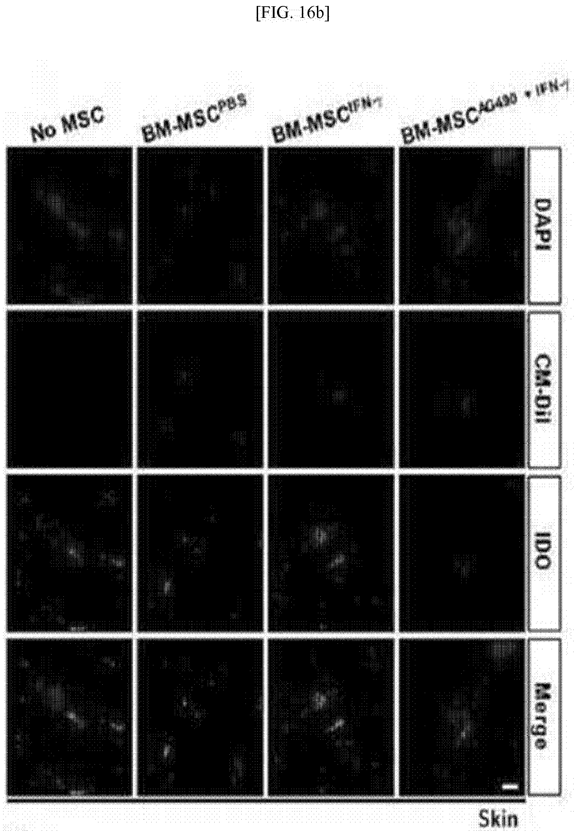

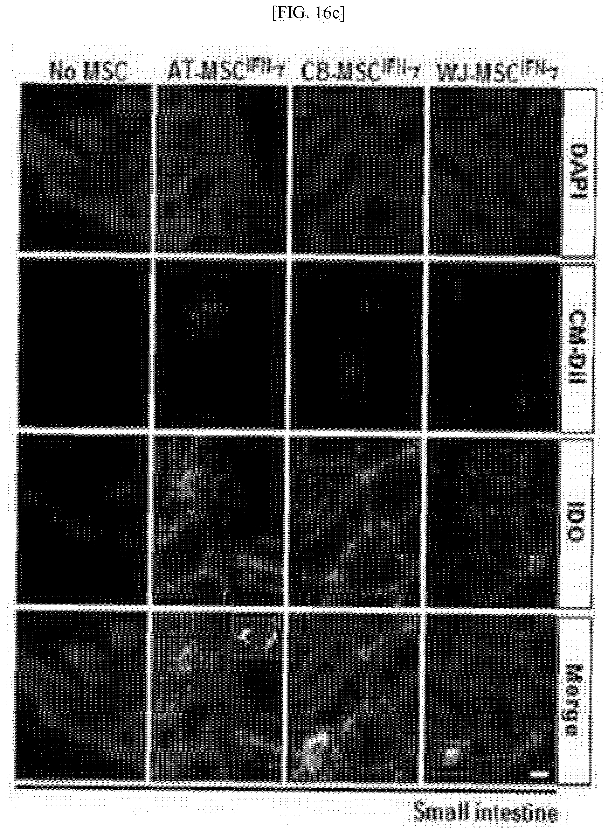

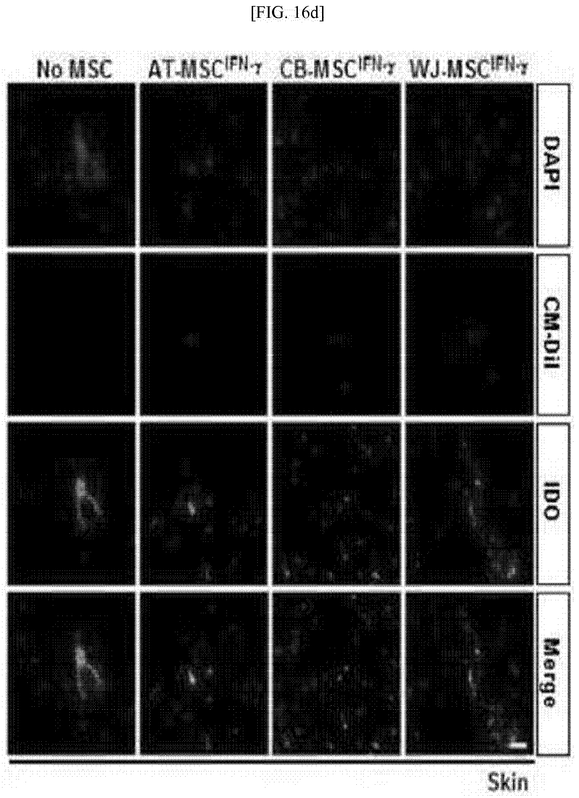

As a result, as shown in FIG. 16, the penetration of the MSCs into tissue was observed, and when the IFN-γ-treated MSCs were injected, it was confirmed that IDO expression was induced in MSCs. However, before the injection of MSCs, when treated with the JAK inhibitor AG490, the penetration of the MSCs into tissue was decreased, and the IDO expression was also decreased.

Based on this result, to examine the IDO role in the immunosuppressive property of the MSCs, they were treated with shRNA inhibiting IDO expression.

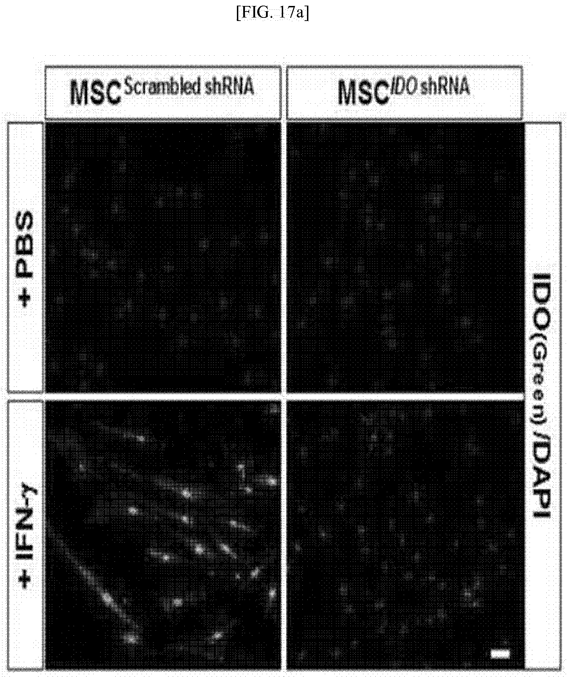

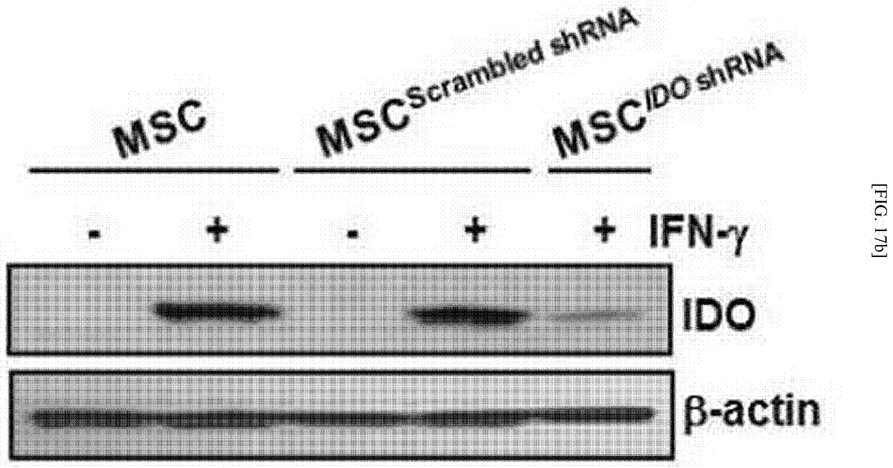

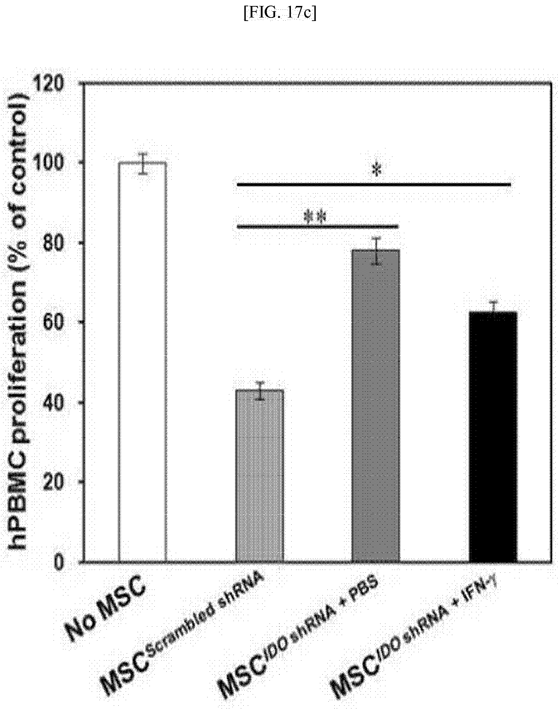

As a result, as shown in FIG. 17, it was confirmed that an IDO expression level was significantly inhibited in the IFN-γ-stimulated MSCs by shRNA targeting *IDO* (FIGS. 17A and 17B), and when PBMCs were co-cultured with PBS or IFN-γ-treated MSCs, PBMC proliferation induced by PHA was significantly recovered, and it was confirmed that IDO expression was inhibited by shRNA targeting *ID* (FIG. 17C).

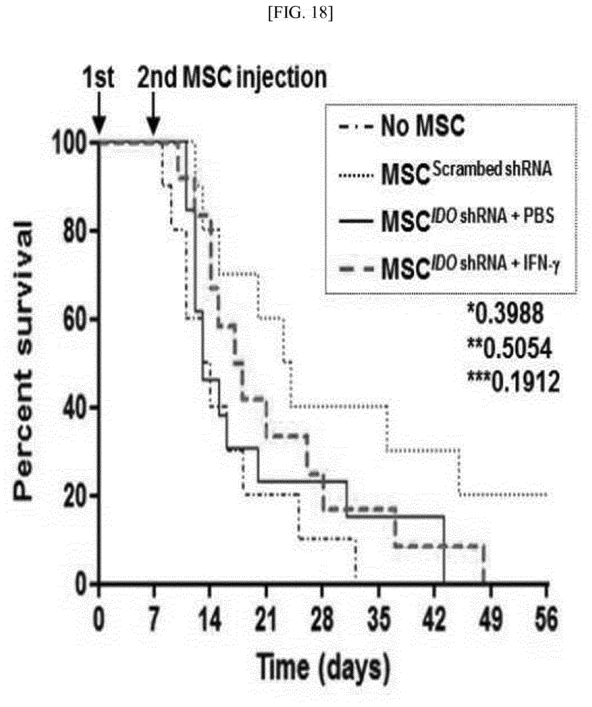

Moreover, IDO expression-decreased MSCs were treated or not treated with IFN-γ, and intravenously administered to a GVHD mouse twice at an interval of 7 days, and therefore, as shown in FIG. 18, there was no significant difference in survival rate in the group into which only PBMCs were injected and a group into which PBMCs were co-injected with IDO-expression-decreased MSCs.

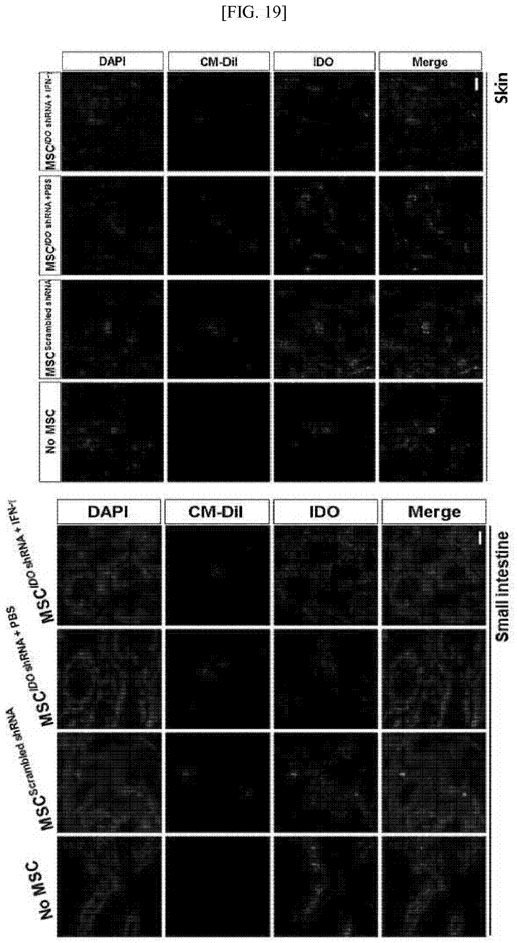

Correlating with the survival outcomes, immunofluorescence images, as shown in FIG. 19, showed that IDO was rarely found in IDO-downregulated MSCs in the small intestine and skin tissues from GVHD mice.

Subsequently, after MSCs stably expressing IDO were established by introducing lentivirus vectors expressing IDO and RFP, the immunosuppressive property of the IDO-overexpressing MSCs was confirmed in *in vitro* and *in vivo* models.

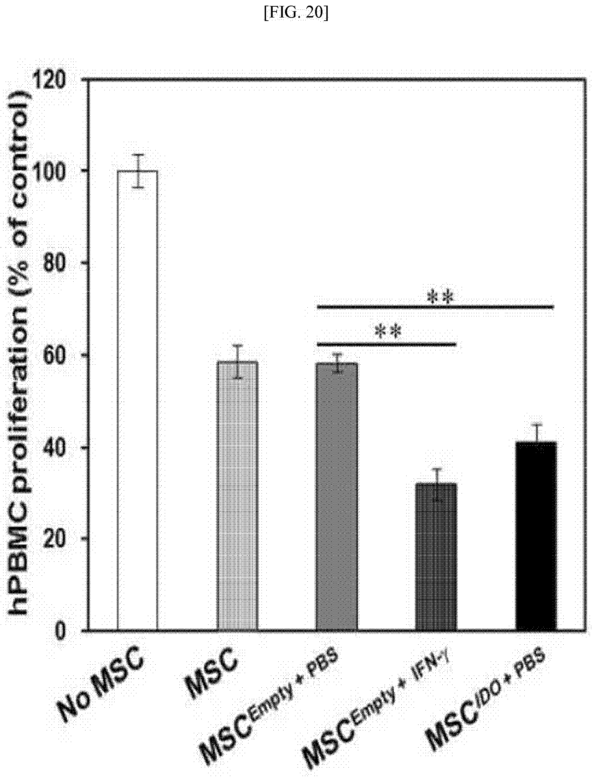

As a result, as shown in FIG. 20, when PBMCs were co-cultured with IDO-overexpressing MSCs, the proliferation of PHA-induced proliferation of PBMCs was significantly inhibited, and this result is similar to the result obtained when PBMCs were co-cultured with IFN-γ-stimulated MSCs.

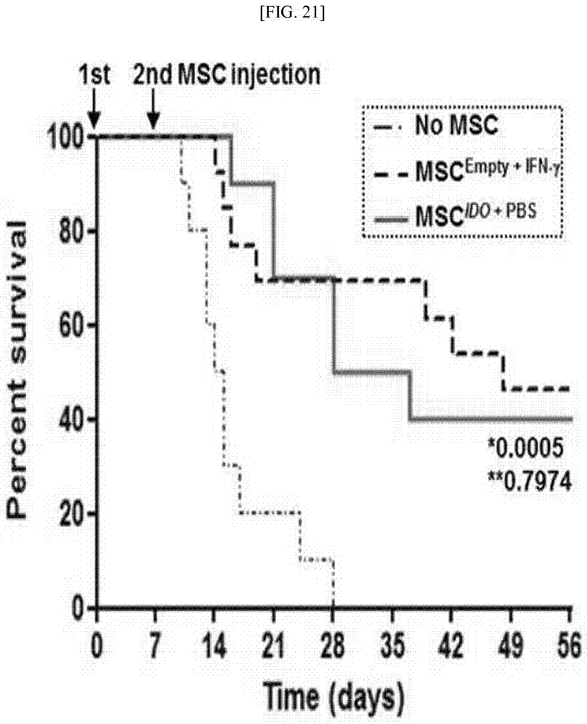

Moreover, it was confirmed that the survival rate of GVHD mice in the IDO-overexpressing MSC group was also increased as much as that shown in the group co-cultured with IFN-γ-treated MSCs (FIG. 21).

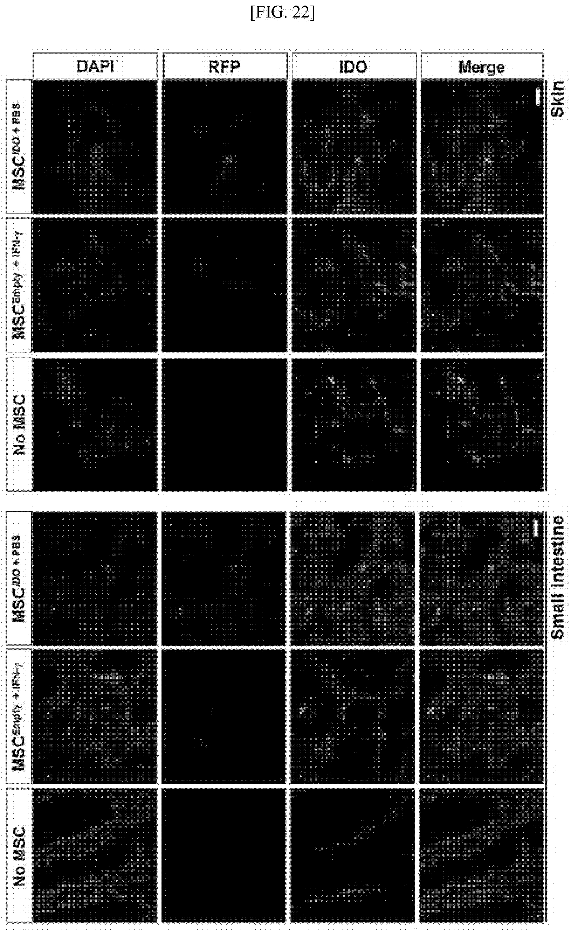

Correlating with the survival rate outcomes, immunofluorescence images, as shown in FIG. 22, showed that IDO was expressed in IDO-overexpressing MSCs in the small intestine and skin tissues of GVHD mice.

Consequently, these results show that the IFN-γ-treated MSCs may home to GVHD-induced tissue, and exhibit the immunosuppressive function through the induction of IDO expression.

### 2-6. IDO expression was induced by IFN-γ stimulation, not by TLR3 activation

Because it has been previously reported that Toll-like receptor 3 (TLR3) induces IDO expression in MSCs for immunosuppressive activity, in this example, immunosuppressive activities between IFN-γ-stimulated MSCs and TLR3-activated MSCs were compared.

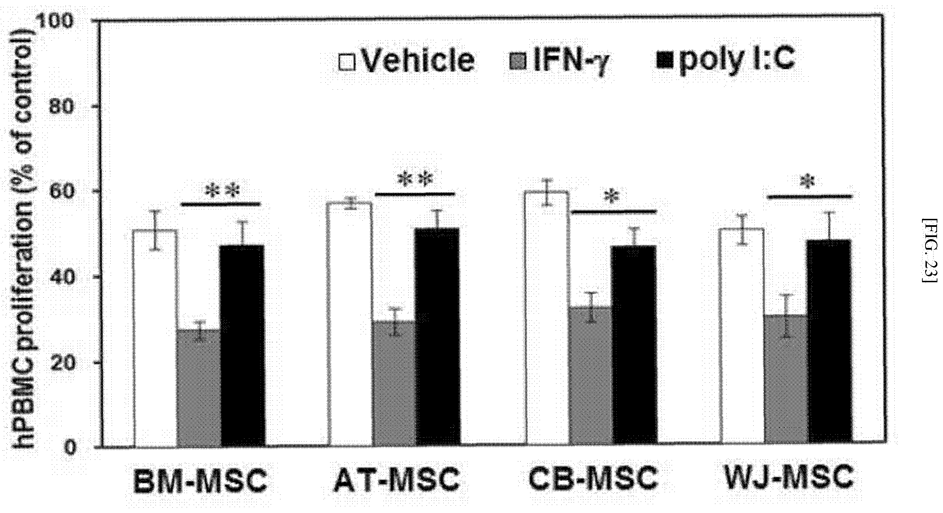

As a result, as shown in FIG. 23, it was confirmed that, when PBMCs were co-cultured with TLR3-activated MSCs by treatment with poly I:C, the proliferation of PBMCs was rarely suppressed, but when PBMCs were co-cultured with IFN-γ-stimulated MSCs derived from bone marrow, adipose tissue, cord blood, and Wharton's jelly, the proliferation of PBMCs was significantly suppressed.

Here, in human MSCs, the induction of IFN-γ expression due to IFN-γ stimulation or TLR3 activation was not observed, and this means that *ex vivo* IFN-γ stimulation is a suitable means for improving the immunosuppressive property of human MSCs.

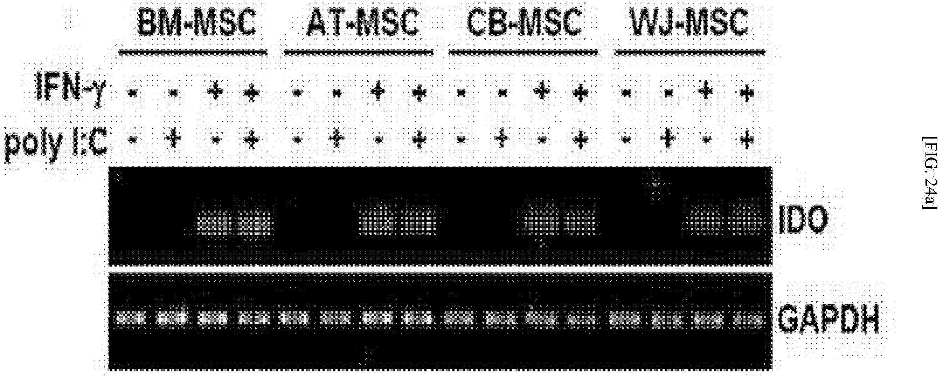

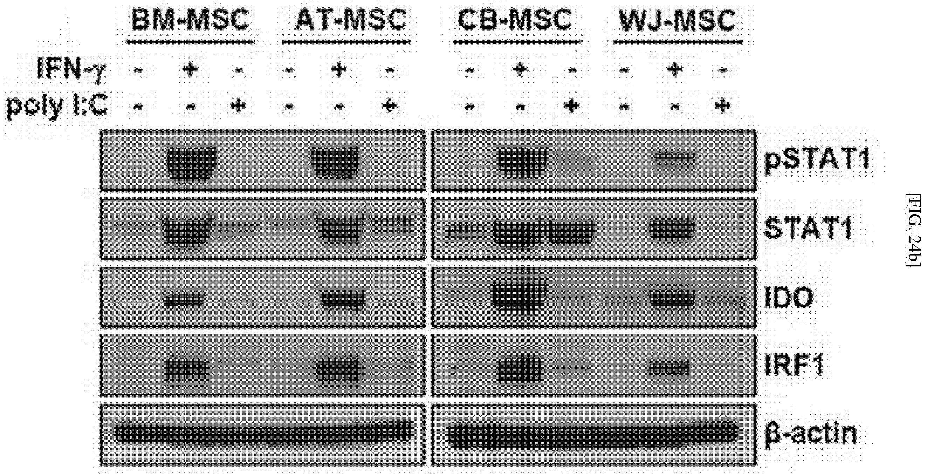

In addition, when the bone-marrow-derived MSCs were stimulated with IFN-γ, IDO expression was induced, but when TLR3 was activated by poly I:C treatment in the MSCs, IDO expression slightly increased, and this result was the same in MSCs derived from various tissues such as adipose tissue, cord blood and Wharton's jelly (FIG. 24).

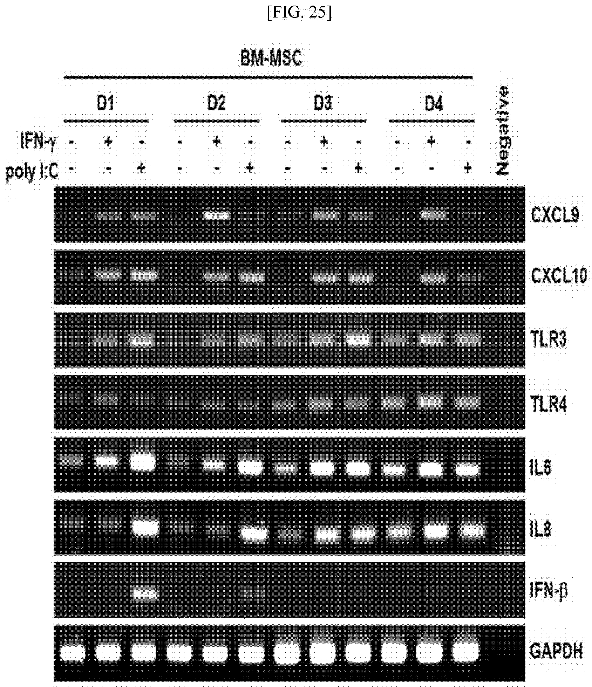

In addition, as shown in FIG. 25, while genes such as *CXCL10, IL-6* and *IL-8* associated with different functions were highly expressed in all of TLR3-activated bone-marrow-derived MSCs, STAT1 activation (STAT1 phosphorylation) was not observed.

According to these results, it can be seen that IFN-γ stimulation induces IDO expression in MSCs (for immunosuppressive activity) through the JAK/STAT1 pathway, but TLR3 activation does not induce IDO expression.

### 2-7. Candidate markers other than IDO biomarker (CXCL9, CXCL10, ICAM2, B7-H1, PTGDS, CXCL11, VCAM1, ICAM1, TRAIL)

RT-PCR was performed to comparatively evaluate the effects on the expression of function genes (IDO, CXCL9, CXCL10, CXCL11, ICAM1, ICAM2, B7-H1, PTGDS, VCAM1 and TRAIL) in adipose-tissue-derived MSCs (AT-MSCs) due to IFN-γ stimulation, TNF-α stimulation and TLR3 activation (poly I:C stimulation).

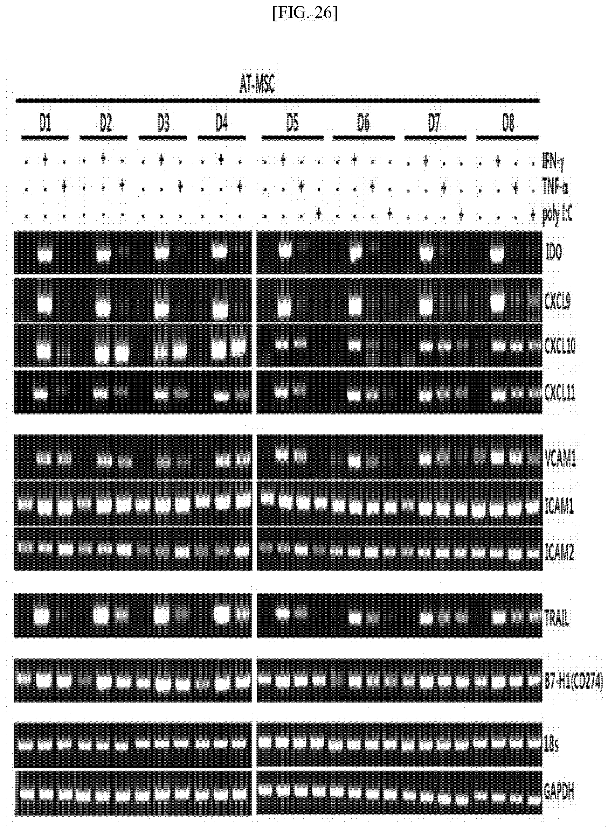

As a result, as shown in FIG. 26, it can be seen that the expression of IDO, CXCL9, CXCL10, CXCL11, ICAM1, ICAM2, B7-H1, PTGDS, VCAM1 and TRAIL was induced specifically by IFN-γ stimulation.

This result means that, to sort highly effective MSCs for treating an immune disorder after stimulation by IFN-γ pretreatment in MSCs, IDO, CXCL9, CXCL10, CXCL11, ICAM1, ICAM2, B7-H1, PTGDS, VCAM1 and TRAIL are effective as immunosuppressive biomarkers.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

The present invention can provide a method useful for acquiring functionally excellent MSCs having an ability to modulate immune responses for clinical treatment of various immune diseases including GVHD and autoimmune diseases, and effectively used as the therapeutic method for immune disorders.

## Claims

1. A method for sorting highly effective mesenchymal stem cells to treat an immune disorder, comprising: measuring the level of an immunosuppressive biomarker after mesenchymal stem cells are stimulated by IFN-γ pretreatment.

2. The method according to claim 1, wherein the method comprises the following steps:
(a) culturing mesenchymal stem cells and treating the cells with IFN-γ;
(b) measuring an expression level of an immunosuppressive biomarker in the mesenchymal stem cells; and
(c) determining the cells in which the expression level is increased compared with an IFN-γ-untreated control as highly effective mesenchymal stem cells for treating an immune disorder.

3. The method according to claim 1, wherein the immunosuppressive biomarker is indoleamine 2,3-dioxygenase (IDO).

4. The method according to claim 3, wherein the immunosuppressive biomarker further includes one or more selected from the group consisting of C-X-C motif ligand 9 (CXCL9), C-X-C motif ligand 10 (CXCL10), C-X-C motif ligand 11 (CXCL11), InterCellular Adhesion Molecule 1 (ICAM1), InterCellular Adhesion Molecule 2 (ICAM2), B7 homolog 1 (B7-H1), Prostaglandin D2 synthase (PTGDS), Vascular Cellular Adhesion Molecule 1 (VCAM1) and TNF-Related Apoptosis-Inducing Ligand (TRAIL).

5. The method according to claim 1, wherein the high efficiency is related to an immunosuppressive property.

6. The method according to claim 1, wherein the mesenchymal stem cells are derived from any one selected from the group consisting of umbilical cord, umbilical cord blood, bone marrow, fat, muscle, Wharton's jelly, nerve, skin, amniotic membrane, chorion, decidua and placenta.

7. The method according to claim 1, wherein the immune disorder is graft-versus-host disease, rejection in organ transplantation, humoral rejection, an autoimmune disease or an allergic disease.

8. The method according to claim 7, wherein the autoimmune disease is Crohn's disease, erythema, atopy, rheumatoid arthritis, Hashimoto's thyroiditis, malignant anemia, Edison's disease, Type I diabetes, lupus, chronic fatigue syndrome, fibromyalgia, hypothyroidism, hyperthyreosis, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, systemic scleroderma, asthma or ulcerative colitis.

9. The method according to claim 3, wherein the IDO expression is increased in IFN-γ-stimulated mesenchymal stem cells via a JAK/STAT1 signaling pathway.

10. The method according to claim 2, wherein the IFN-γ in the step (a) is contained in a medium at a concentration of 1 to 100 IU/mL.

11. The method according to claim 2, wherein an expression level of the biomarker in the step (b) is measured using western blotting, antibody immunoprecipitation, ELISA, mass spectrometry, RT-PCR, competitive RT-PCR, real-time RT-PCR, RNase protection assay (RPA), northern blotting or a DNA chip.

12. Highly effective mesenchymal stem cells for treating an immune disorder, which are sorted by the method of claim 1.

13. The cells according to claim 12, wherein the immune disorder is graft-versus-host disease, rejection in organ transplantation, humoral rejection, an autoimmune disease or an allergic disease.

14. The cells according to claim 12, wherein the high efficiency is related to an immunosuppressive property.

15. The cells according to claim 12, wherein the mesenchymal stem cells are derived from umbilical cord, umbilical cord blood, bone marrow, fat, muscle, Wharton's jelly, nerve, skin, amniotic membrane or placenta.

16. The cells according to claim 12, wherein the mesenchymal stem cells are derived from autologous, xenogeneic or allogeneic cells.

17. A pharmaceutical composition for treating an immune disorder, which contains the highly effective mesenchymal stem cells of claim 12.

18. A pharmaceutical preparation for treating graft-versus-host disease, which contains the highly effective mesenchymal stem cells of claim 12.

19. A method for treating an immune disorder, comprising:
administering the highly effective mesenchymal stem cells of claim 12 to a subject.

20. A use of the highly effective mesenchymal stem cells of claim 12 for treating an immune disorder.
